# EUROPEAN PATENT SPECIFICATION

(11) **EP 2 976 422 B1**
(45) Date of publication and mention of the grant of the patent: **13.12.2017**
(21) Application number: 14717171.4
(22) Date of filing: 18.03.2014
(51) Int. Cl.: C12N 5/00

(54) **CO-EXPRESSION OF FACTOR VIII AND VON WILLEBRAND FACTOR**
GEMEINSAME EXPRESSION VON FAKTOR VIII UND DEM VON-WILLEBRAND-FAKTOR
CO-EXPRESSION DU FACTEUR VIII ET DU FACTEUR DE VON WILLEBRAND

(30) Priority: 19.03.2013 GB 201304973
(43) Date of publication of application: 27.01.2016
(73) Proprietor: Profactor Pharma Ltd., Roslin, Midlothian EH25 9PP (GB)
(72) Inventor: MCVEY, John Henderson, Beaconsfield HP9 2TU (GB); GARNER, Ian, Edinburgh EH8 7DW (GB); DALE, Adele, Midlothian EH25 9PP (GB); VERNON, Bruce, Edinburgh EH10 7EG (GB)
(74) Representative: Chapman, Paul Gilmour
(86) International application number: PCT/GB2014/050850
(87) International publication number: WO 2014/147386

(56) References cited:
- WO-A2-2011/060242
- SHOVLIN CLAIRE L ET AL: "Endothelial cell processing and alternatively spliced transcripts of factor VIII: potential implications for coagulation cascades and pulmonary hypertension.", PLOS ONE 2010, vol. 5, no. 2, E9154, 2010, pages 1-9, XP002727998, ISSN: 1932-6203
- WARD NATALIE J ET AL: "Codon optimization of human factor VIII cDNAs leads to high-level expression", BLOOD, vol. 117, no. 3, January 2011 (2011-01), pages 798-807, XP055052195,
- McIntosh et al.: "Therapeutic levels of FVIII following a single peripheral vein administration of rAAV vector encoding a novel human factor VIII variant", Internet Blood, vol. 121, no. 17 20 February 2013 (2013-02-20), pages 3335-3344, XP002727999, ISSN: 1528-0020, DOI: 10.1182/blood-2012-10-462200 Retrieved from the Internet: URL:http://www.bloodjournal.org/content/12 1/17/3335 [retrieved on 2014-07-29]
- CHEN CHUN ET AL: "The gene expression of coagulation factor VIII in mammalian cell lines", THROMBOSIS RESEARCH, vol. 95, no. 2, 15 July 1999 (1999-07-15), pages 105-115, XP002728000, ISSN: 0049-3848
- PIPE S W ET AL: "Functional factor VIII made with von Willebrand factor at high levels in transgenic milk", JOURNAL OF THROMBOSIS AND HAEMOSTASIS, vol. 9, no. 11, 1 November 2011 (2011-11-01), pages 2235-2242, XP002683522, WILEY INTERSCIENCE, ENGLAND ISSN: 1538-7836, DOI: 10.1111/J.1538-7836.2011.04505.X [retrieved on 2011-11-01]
- KAUFMAN RJ ET AL: "Effect of von Willebrand Factor coexpression on the synthesis and secretion of Factor VIII in Chinese Hamster Ovary Cells", MOLECULAR AND CELLULAR BIOLOGY, AMERICAN SOCIETY FOR MICROBIOLOGY, WASHINGTON, US, vol. 9, no. 3, 1 January 1989 (1989-01-01) , pages 1233-1242, XP002140918, ISSN: 0270-7306

### SEQUENCE MISTING

<110> Profactor Pharma Ltd
<120> Recombinant Protein
<130> PE955669WO
<150> GB1304973.9
   <151> 2013-03-19
<160> 4
<170> PatentIn version 3.5
<210> 1
   <211> 4377
   <212> DNA
   <213> Artificial Sequence
<220>
   <223> codon optimised B domain deleted F VIII sequence for expression in CHO cells
<400> 1
   atgcagatcg agctgtccac ctgctttttt ctgtgcctgc tgcggttctg cttcagcgcc      60
   acccggcggt actacctggg cgccgtggag ctgtcctggg actacatgca gagcgacctg     120
   ggcgagctgc ctgtggacgc ccggttcccc cccagagtgc ccaagagctt ccccttcaac     180
   accagcgtgg tgtacaagaa aaccctgttc gtggagttca ccgaccacct gttcaatatc     240
   gccaagccca ggcccccctg gatgggcctg ctgggcccca ccatccaggc cgaggtgtac     300
   gacaccgtgg tgatcaccct gaagaacatg gccagccacc ccgtgagcct gcacgccgtg     360
   ggcgtgagct actggaaggc cagcgagggc gccgagtacg acgaccagac cagccagcgg     420
   gagaaagaag atgacaaggt gttccctggc ggcagccaca cctacgtgtg gcaggtgctg     480
   aaagaaaacg gccccatggc ctccgacccc ctgtgcctga cctacagcta cctgagccac     540
   gtggacctgg tgaaggacct gaacagcggc ctgatcggcg ctctgctcgt ctgccgggag     600
   ggcagcctgg ccaaagagaa aacccagacc ctgcacaagt tcatcctgct gttcgccgtg     660
   ttcgacgagg gcaagagctg gcacagcgag acaaagaaca gcctgatgca ggaccgggac     720
   gccgcctctg ccagagcctg gcccaagatg cacaccgtga acggctacgt gaacagaagc     780
   ctgcccggcc tgattggctg ccaccggaag agcgtgtact ggcacgtgat cggcatgggc     840
   accacacccg aggtgcacag catctttctg gaagggcaca cctttctggt ccggaaccac     900
   cggcaggcca gcctggaaat cagccctatc accttcctga ccgcccagac actgctgatg     960
   gacctgggcc agttcctgct gttttgccac atcagctctc accagcacga cggcatggaa    1020
   gcctacgtga aggtggactc ttgccccgag gaaccccagc tgcggatgaa gaacaacgag    1080
   gaagccgagg actacgacga cgacctgacc gacagcgaga tggacgtggt gcggttcgac    1140
   gacgacaaca gccccagctt catccagatc agaagcgtgg ccaagaagca ccccaagacc 1200
   tgggtgcact atatcgccgc cgaggaagag gactgggact acgcccccct ggtgctggcc 1260
   cccgacgaca gaagctacaa gagccagtac ctgaacaatg gcccccagcg gatcggccgg 1320
   aagtacaaga aagtgcggtt catggcctac accgacgaga cattcaagac ccgggaggcc 1380
   atccagcacg agagcggcat cctgggcccc ctgctgtacg gcgaagtggg cgacacactg 1440
   ctgatcatct tcaagaacca ggctagccgg ccctacaaca tctaccccca cggcatcacc 1500
   gacgtgcggc ccctgtacag caggcggctg cccaagggcg tgaagcacct gaaggacttc 1560
   cccatcctgc ccggcgagat cttcaagtac aagtggaccg tgaccgtgga ggacggcccc 1620
   accaagagcg accccagatg cctgacccgg tactacagca gcttcgtgaa catggaacgg 1680
   gacctggcct ccgggctgat cggacctctg ctgatctgct acaaagaaag cgtggaccag 1740
   cggggcaacc agatcatgag cgacaagcgg aacgtgatcc tgttcagcgt gttcgatgag 1800
   aaccggtcct ggtatctgac cgagaacatc cagcggtttc tgcccaaccc tgccggcgtg 1860
   cagctggaag atcccgagtt ccaggccagc aacatcatgc actccatcaa tggctacgtg 1920
   ttcgactctc tgcagctctc cgtgtgtctg cacgaggtgg cctactggta catcctgagc 1980
   atcggcgccc agaccgactt cctgagcgtg ttcttcagcg gctacacctt caagcacaag 2040
   atggtgtacg aggacaccct gaccctgttc cctttcagcg gcgagacagt gttcatgagc 2100
   atggaaaacc ccggcctgtg gattctgggc tgccacaaca gcgacttccg gaaccggggc 2160
   atgaccgccc tgctgaaggt gtccagctgc gacaagaaca ccggcgacta ctacgaggac 2220
   agctacgagg atatcagcgc ctacctgctg tccaagaaca acgccatcga accccggagc 2280
   ttcagccaga acccccccgt gctgaagcgt caccagcggg agatcacccg gacaaccctg 2340
   cagtccgacc aggaagagat cgattacgac gacaccatca gcgtggagat gaagaaagag 2400
   gatttcgata tctacgacga ggacgagaac cagagcccca gaagcttcca gaagaaaacc 2460
   cggcactact tcattgccgc cgtggagagg ctgtgggact acggcatgag ttctagcccc 2520
   cacgtgctgc ggaaccgggc ccagagcggc agcgtgcccc agttcaagaa agtggtgttc 2580
   caggaattca cagacggcag cttcacccag cctctgtata gaggcgagct gaacgagcac 2640
   ctggggctgc tggggcccta catcagggcc gaagtggagg acaacatcat ggtgaccttc 2700
   cggaatcagg ccagcagacc ctactccttc tacagcagcc tgatcagcta cgaagaggac 2760
   cagcggcagg gcgccgaacc ccggaagaac ttcgtgaagc ccaacgaaac caagacctac 2820
   ttctggaaag tgcagcacca catggccccc accaaggacg agttcgactg caaggcctgg 2880
   gcctacttca gcgacgtgga tctggaaaag gacgtgcact ctggactgat tggcccactc 2940
   ctggtctgcc acactaacac cctcaacccc gcccacggcc gccaggtgac cgtgcaggaa 3000
   ttcgccctgt tcttcaccat cttcgacgag acaaagtcct ggtacttcac cgagaatatg 3060
   gaacggaact gcagagcccc ctgcaacatc cagatggaag atcctacctt caaagagaac 3120
   taccggttcc acgccatcaa cggctacatc atggacaccc tgcctggcct ggtgatggcc 3180
   caggaccaga gaatccggtg gtatctgctg tccatgggca gcaacgagaa tatccacagc 3240
   atccacttca gcggccacgt gttcaccgtg cggaagaaag aagagtacaa gatggccctg 3300
   tacaacctgt accccggcgt gttcgagaca gtggagatgc tgcccagcaa ggccggcatc 3360
   tggcgggtgg agtgtctgat cggcgagcac ctgcacgctg gcatgagcac cctgtttctg 3420
   gtgtacagca acaagtgcca gaccccactg ggcatggcct ctggccacat ccgggacttc 3480
   cagatcaccg cctccggcca gtacggccag tgggccccca agctggccag actgcactac 3540
   agcggcagca tcaacgcctg gtccaccaaa gagcccttca gctggatcaa ggtggacctg 3600
   ctggccccta tgatcatcca cggcattaag acccagggcg ccaggcagaa gttcagcagc 3660
   ctgtacatca gccagttcat catcatgtac agcctggacg gcaagaagtg gcagacctac 3720
   cggggcaaca gcaccggcac cctgatggtg ttcttcggca atgtggacag cagcggcatc 3780
   aagcacaaca tcttcaaccc ccccatcatt gcccggtaca tccggctgca ccccacccac 3840
   tacagcatta gatccacact gagaatggaa ctgatgggct gcgacctgaa ctcctgcagc 3900
   atgcctctgg gcatggaaag caaggccatc agcgacgccc agatcacagc cagcagctac 3960
   ttcaccaaca tgttcgccac ctggtccccc tccaaggcca ggctgcacct gcagggccgg 4020
   tccaacgcct ggcggcctca ggtcaacaac cccaaagaat ggctgcaggt ggactttcag 4080
   aaaaccatga aggtgaccgg cgtgaccacc cagggcgtga aaagcctgct gaccagcatg 4140
   tacgtgaaag agtttctgat cagcagctct caggatggcc accagtggac cctgttcttt 4200
   cagaacggca aggtgaaagt gttccagggc aaccaggact ccttcacccc cgtggtgaac 4260
   tccctggacc cccccctgct gacccgctac ctgagaatcc acccccagtc ttgggtgcac 4320
   cagatcgccc tcaggatgga agtcctggga tgtgaggccc aggatctgta ctgatga 4377
<210> 2
   <211> 8445
   <212> DNA
   <213> Artificial Sequence
<220>
   <223> codon optimised VWF sequence for CHO expression
<400> 2
   atgatccctg ccagatttgc cggcgtgctg ctggccctgg ctctgatcct gcctggcaca      60
   ctgtgtgccg agggcaccag aggcagatcc tctaccgccc ggtgttccct gttcggctcc     120
   gacttcgtga acaccttcga cggctccatg tacagcttcg ccggctactg ctcctacctg     180
   ctggctggcg gctgccagaa gcggtccttc tctatcatcg gcgacttcca gaacggcaag     240
   cgggtgtccc tgtccgtgta cctgggcgag ttcttcgaca tccacctgtt cgtgaacggc 300
   accgtgaccc agggcgatca gagggtgtcc atgccctacg cctccaaggg cctgtacctg 360
   gaaaccgagg ccggctacta caagctgtcc ggcgaggcct acggcttcgt ggccagaatc 420
   gatggctccg gcaacttcca ggtgctgctg tccgaccggt acttcaacaa gacctgcggc 480
   ctgtgcggca acttcaacat cttcgccgag gacgacttca tgacccagga aggcaccctg 540
   acctccgacc cctacgactt cgccaactcc tgggctctgt cctccggcga gcagtggtgc 600
   gagagagcca gccctccttc ctcctcctgc aacatctcca gcggcgagat gcagaaaggc 660
   ctgtgggagc agtgccagct gctgaagtcc acctctgtgt tcgcccggtg ccaccctctg 720
   gtggaccctg agccttttgt ggccctgtgc gaaaagaccc tgtgcgagtg tgctggcggc 780
   ctggaatgtg cctgtcctgc cctgctggaa tacgccagaa cctgcgctca ggaaggcatg 840
   gtcctgtacg gctggaccga ccactctgcc tgctctccag tgtgtcctgc cggcatggaa 900
   taccggcagt gcgtgtcccc ttgcgccagg acctgtcagt ccctgcacat caacgagatg 960
   tgccaggaac gctgcgtgga cggctgctct tgtcctgagg gacagctgct ggacgagggc 1020
   ctgtgcgtgg aatctaccga gtgcccttgc gtgcactccg gcaagagata ccctcctggc 1080
   acctccctgt cccgggactg caacacctgt atctgccgga actcccagtg gatctgctcc 1140
   aacgaggaat gccctggcga gtgcctggtc accggccagt cccacttcaa gtccttcgac 1200
   aacagatact tcaccttctc cggcatctgc cagtatctgc tggccagaga ctgccaggac 1260
   cactccttct ccatcgtgat cgaaaccgtg cagtgcgccg acgacagaga tgccgtgtgc 1320
   accagatctg tgaccgtgcg gctgcctggc ctgcacaact ctctggtcaa gctgaagcac 1380
   ggcgctggcg tggccatgga tggacaggat gtgcagctgc ccctgctgaa gggcgacctg 1440
   agaatccagc acaccgtgac cgcaagcgtg cggctgtctt acggcgagga cctgcagatg 1500
   gactgggacg gcagaggcag actgctggtc aaactgtccc ctgtgtacgc cggcaagacc 1560
   tgtggactgt gtggaaacta caacggcaac cagggcgacg atttcctgac cccttctggc 1620
   ctggccgagc cccgggtgga agattttggc aacgcctgga agctgcacgg cgactgtcag 1680
   gatctgcaga agcagcactc cgacccttgc gccctgaacc cccggatgac ccggttttct 1740
   gaagaggcct gtgccgtgct gacctcccct acctttgagg cctgtcacag agccgtgtcc 1800
   cccctgccct acctgcggaa ctgtagatac gacgtgtgct cctgctccga cggccgcgaa 1860
   tgtctgtgtg gcgccctggc ttcttacgcc gctgcttgtg ctggcagagg cgtcagagtg 1920
   gcttggcggg aacctggcag atgcgagctg aactgcccta agggccaggt gtacctgcag 1980
   tgcggcaccc cttgcaacct gacctgtcgg agcctgtcct accccgacga agagtgcaac 2040
   gaggcctgcc tggaaggctg cttctgtccc cctggcctgt acatggacga gaggggcgac 2100
   tgtgtgccca aggcccagtg cccctgttac tacgacggcg agatcttcca gcccgaggac 2160
   atcttctccg accaccacac catgtgctac tgcgaggatg gcttcatgca ctgcaccatg 2220
   tctggcgtgc ccggctctct gctgcctgat gctgtgctgt ccagccctct gtcccaccgg 2280
   tccaagagat ccctgtcttg ccggcctccc atggtcaagc tggtctgccc tgccgacaac 2340
   ctgagagccg agggactgga atgcaccaag acctgccaga actacgacct ggaatgcatg 2400
   tccatgggct gcgtgtccgg ctgcctgtgt ccacctggaa tggtccgaca cgagaacaga 2460
   tgcgtggccc tggaacggtg cccatgcttt catcagggca aagagtacgc ccctggcgag 2520
   acagtgaaga tcggctgcaa tacctgcgtg tgccgggacc ggaagtggaa ctgcaccgat 2580
   cacgtgtgcg acgccacctg ttccacaatc ggcatggccc actacctgac cttcgatggc 2640
   ctgaagtacc tgttccccgg cgagtgccag tacgtgctgg tgcaggacta ctgcggctcc 2700
   aaccccggca ccttcagaat cctggtcgga aacaagggct gctcccaccc ctccgtgaag 2760
   tgcaagaagc gggtcaccat cctggtcgag ggcggcgaga tcgagctgtt cgacggcgaa 2820
   gtgaacgtga agcggcccat gaaggacgag acacacttcg aggtggtgga atccggccgg 2880
   tacatcatcc tgctgctggg caaggccctg tccgtcgtgt gggacagaca cctgagcatc 2940
   tccgtggtgc tgaagcagac ctaccaggaa aaagtctgcg ggctgtgtgg caatttcgac 3000
   ggcatccaga acaacgatct gacctccagc aacctgcagg tcgaagagga ccccgtggac 3060
   ttcggcaaca gctggaaggt gtcctcccag tgtgccgaca ccagaaaggt gcccctggac 3120
   tcctctcccg ccacctgtca caacaacatc atgaagcaga caatggtgga ctcctcctgt 3180
   agaatcctga ccagcgacgt gttccaggac tgtaacaagc tggtcgatcc cgagccctac 3240
   ctggacgtgt gtatctacga tacctgctcc tgcgagtcta tcggcgactg cgcctgcttc 3300
   tgcgacacaa tcgccgccta cgcccacgtg tgcgctcagc atggaaaggt ggtcacctgg 3360
   cggaccgcca ccctgtgtcc tcagtcctgc gaggaacgga acctgcgcga gaacggctac 3420
   gagtgcgagt ggcggtacaa ctcttgcgcc cctgcctgcc aggtcacatg ccagcatcct 3480
   gagcctctgg cctgccctgt gcagtgcgtg gaaggctgtc acgcccattg tcctcccggc 3540
   aagatcctgg acgagctgct gcagacctgt gtggaccccg aggactgtcc tgtgtgcgaa 3600
   gtggccggca gaagattcgc cagcggcaag aaagtgaccc tgaacccttc cgaccccgag 3660
   cactgccaga tctgccactg cgacgtggtc aatctgacct gcgaggcttg ccaggaacca 3720
   ggcggactgg tcgtgcctcc taccgatgct cctgtgtccc ccaccaccct gtacgtcgag 3780
   gacatcagcg agccccccct gcacgacttc tactgctcta ggctgctgga cctggtgttc 3840
   ctgctggacg gctcctccag actgtccgag gccgagttcg aggtgctgaa ggccttcgtg 3900
   gtggacatga tggaacggct gcggatctcc cagaaatggg tccgagtggc cgtggtggaa 3960
   taccacgatg gctcccacgc ctacatcggc ctgaaggaca gaaagcggcc ttccgagctg 4020
   cggcggatcg ccagccaggt caaatacgct ggaagccagg tggcctccac ctccgaagtg 4080
   ctgaagtata ccctgttcca gatcttctcc aagatcgacc ggcccgaggc ctccagaatc 4140
   acactgctgc tgatggcctc ccaggaaccc cagcggatgt cccggaactt cgtcagatac 4200
   gtgcagggcc tgaagaaaaa gaaagtgatc gtgatccccg tgggcatcgg cccccacgcc 4260
   aatctgaagc agatccggct gatcgagaag caggcccccg agaacaaggc ctttgtgctg 4320
   tcctccgtgg atgagctgga acagcagcgg gacgagatcg tgtcctacct gtgcgatctg 4380
   gcccctgagg ctcctcctcc taccctgcct cctgatatgg cccaggtcac agtgggacct 4440
   ggcctgctgg gagtgtctac cctgggaccc aagcggaaca gcatggtgct ggacgtggca 4500
   ttcgtgctgg aaggctccga caagatcggc gaggccgact tcaaccggtc caaagagttc 4560
   atggaagagg tcatccagcg gatggacgtg ggccaggact ccatccacgt gaccgtgctg 4620
   cagtactcct acatggtcac cgtggaatac cccttcagcg aggcccagtc caagggcgac 4680
   atcctgcaga gagtgcgcga gatcagatac cagggcggca acaggaccaa caccggcctg 4740
   gcactgagat acctgtccga ccacagtttt ctggtgtctc agggcgacag agagcaggcc 4800
   cctaacctgg tgtatatggt caccggaaac cccgcctccg acgagatcaa gagactgccc 4860
   ggcgacatcc aggtggtgcc tatcggcgtg ggacccaacg ccaacgtgca ggaactggaa 4920
   cggatcggct ggcccaacgc ccccatcctg atccaggact tcgagacact gccccgcgag 4980
   gcccctgatc tggtgctgca gagatgctgt agcggcgagg ggctgcagat ccctaccctg 5040
   tctccagccc ctgactgctc ccagcctctg gacgtgatcc tgctgctgga cggcagctct 5100
   agcttccccg cctcctactt cgacgagatg aagtccttcg ccaaggcctt catctccaag 5160
   gccaacatcg gccctcggct gacccaggtg tccgtcctgc agtacggctc catcaccacc 5220
   atcgacgtgc catggaacgt ggtgcccgag aaggcccatc tgctgtccct ggtggacgtg 5280
   atgcagagag agggcggacc ttcccagatc ggagatgccc tgggcttcgc cgtgcggtat 5340
   ctgacctctg agatgcacgg cgccagacct ggcgcctcta aggctgtggt catcctggtc 5400
   acagacgtgt ccgtggacag cgtggacgct gccgctgatg ccgccagatc caacagagtg 5460
   accgtgttcc ctatcggcat cggcgacaga tacgacgccg cccagctgag aatcctggct 5520
   ggacctgccg gcgactccaa cgtcgtgaag ctgcagagaa tcgaggatct gcccaccatg 5580
   gtgaccctgg gcaacagctt cctgcacaag ctgtgctccg gcttcgtgcg gatctgcatg 5640
   gacgaggacg gcaacgagaa gcggcctggc gacgtgtgga cactgcctga tcagtgccat 5700
   accgtgacct gccagcctga cggccagacc ctgctgaaat cccacagagt gaactgcgac 5760
   cggggcctga ggccttcctg ccctaactct cagtcccccg tgaaggtgga agagacatgc 5820
   ggctgccggt ggacctgccc ttgtgtgtgt accggctcct ctacccggca catcgtgacc 5880
   tttgacggac agaacttcaa gctgaccggc tcctgtagct acgtgctgtt ccagaacaaa 5940
   gaacaggacc tggaagtgat tctgcacaac ggcgcctgct ccccaggcgc tagacagggc 6000
   tgcatgaagt ctatcgaagt gaagcactcc gccctgagcg tcgagctgca ctccgacatg 6060
   gaagtgaccg tgaatggccg gctggtgtcc gtgccttacg tgggcggcaa tatggaagtg 6120
   aatgtgtacg gcgccatcat gcacgaagtg cggttcaacc acctgggcca catcttcacc 6180
   ttcacacctc agaacaatga gttccagctg cagctgtccc ccaagacctt cgcctccaag 6240
   acatacggcc tgtgtggaat ctgtgacgag aacggcgcca acgatttcat gctgcgggac 6300
   ggaaccgtga ccaccgactg gaaaaccctg gtccaggaat ggaccgtcca gcgcccagga 6360
   cagacctgtc agcccatcct ggaagaacag tgcctggtgc ccgactccag ccactgtcag 6420
   gtcctgctgc tgcctctgtt cgccgagtgc cacaaggtgc tggcccctgc caccttctac 6480
   gctatctgtc agcaggacag ctgccaccag gaacaggtct gcgaagtcat tgccagctac 6540
   gctcacctgt gccggaccaa cggcgtgtgt gtggattggc ggacccctga cttctgcgcc 6600
   atgtcctgtc ccccttccct ggtgtacaac cactgcgagc acggctgccc cagacactgt 6660
   gatggcaacg tgtcctcctg cggcgaccac ccttctgagg gctgtttctg ccctcctgac 6720
   aaagtgatgc tggaaggatc ttgcgtgccc gaagaggctt gcacccagtg catcggagag 6780
   gatggcgtgc agcaccagtt tctggaagcc tgggtgccag accaccagcc ttgtcagatc 6840
   tgcacctgtc tgtccggccg gaaagtgaac tgtaccaccc agccttgccc caccgccaag 6900
   gctcctacat gtggcctgtg tgaagtggcc cggctgagac agaacgccga ccagtgctgt 6960
   cccgagtacg agtgcgtgtg cgaccccgtg tcttgcgatc tgcctcctgt gccccattgc 7020
   gagagaggcc tgcagcctac cctgaccaac ccaggcgagt gccggcccaa ctttacctgc 7080
   gcctgccgga aagaggaatg caagagagtc agccccccca gctgcccccc tcatagactg 7140
   ccaaccctga gaaagaccca gtgctgtgat gagtatgagt gcgcctgcaa ctgcgtgaac 7200
   tccaccgtgt cctgtcctct gggctacctg gccagcaccg ccaccaacga ttgtggctgc 7260
   accaccacca cctgtctgcc cgacaaagtg tgcgtgcacc ggtccaccat ctaccccgtg 7320
   ggccagtttt gggaagaggg ctgcgacgtg tgtacctgta ccgatatgga agatgccgtg 7380
   atgggcctgc gggtggccca gtgttctcag aagccttgcg aggactcttg cagatctggc 7440
   ttcacctacg tgctgcacga gggcgagtgc tgcggcagat gtctgccttc tgcttgcgag 7500
   gtcgtgaccg gctctcctag aggcgactcc cagtcctcct ggaagtccgt gggatctcag 7560
   tgggcctccc ctgagaaccc ttgcctgatc aacgagtgtg tgcgcgtgaa agaagaggtg 7620
   ttcatccagc agcggaacgt gtcctgcccc cagctggaag tccctgtgtg cccttccggc 7680
   ttccagctga gttgcaagac ctccgcctgc tgcccttcct gtagatgcga gcggatggaa 7740
   gcctgcatgc tgaatggcac agtgatcggc cctggcaaga ccgtgatgat tgacgtgtgc 7800
   acaacctgcc ggtgcatggt gcaggtcgga gtgatcagcg gcttcaagct ggaatgcaga 7860
   aagaccacct gtaacccctg ccccctgggc tacaaagaag agaacaacac cggcgagtgt 7920
   tgtggccggt gcctgcctac cgcttgcacc attcagctga gaggcggcca gatcatgacc 7980
   ctgaagcggg acgaaaccct gcaggacggc tgcgataccc acttctgcaa agtgaacgag 8040
   cggggcgagt acttctggga gaagagagtg accggctgcc ccccattcga cgagcacaag 8100
   tgtctggctg agggcggaaa gattatgaag atccctggca cctgttgcga cacatgcgag 8160
   gaacccgagt gtaacgacat caccgccaga ctgcagtacg tgaaagtggg ctcctgcaag 8220
   tccgaggtgg aagtggacat ccactactgc cagggcaagt gcgccagcaa ggccatgtac 8280
   tccatcgaca tcaacgacgt gcaggaccag tgctcttgct gctcccccac cagaaccgag 8340
   cctatgcagg tcgccctgca ctgtaccaac ggctccgtgg tgtaccacga ggtcctgaac 8400
   gccatggaat gcaagtgctc cccccggaag tgctccaagt gatga 8445
<210> 3
   <211> 1457
   <212> PRT
   <213> Homo sapiens
<400> 3
   Met Gln Ile Glu Leu Ser Thr Cys Phe Phe Leu Cys Leu Leu Arg Phe
   1               5                   10                  15
   Cys Phe Ser Ala Thr Arg Arg Tyr Tyr Leu Gly Ala Val Glu Leu Ser
               20                  25                  30
   Trp Asp Tyr Met Gln Ser Asp Leu Gly Glu Leu Pro Val Asp Ala Arg
           35                  40                  45
   Phe Pro Pro Arg Val Pro Lys Ser Phe Pro Phe Asn Thr Ser Val Val
       50                  55                  60
   Tyr Lys Lys Thr Leu Phe Val Glu Phe Thr Asp His Leu Phe Asn Ile
   65                  70                  75                  80
   Ala Lys Pro Arg Pro Pro Trp Met Gly Leu Leu Gly Pro Thr Ile Gln
                   85                  90                  95
   Ala Glu Val Tyr Asp Thr Val Val Ile Thr Leu Lys Asn Met Ala Ser
               100                 105                 110
   His Pro Val Ser Leu His Ala Val Gly Val Ser Tyr Trp Lys Ala Ser
           115                 120                 125
   Glu Gly Ala Glu Tyr Asp Asp Gln Thr Ser Gln Arg Glu Lys Glu Asp
       130                 135                 140
   Asp Lys Val Phe Pro Gly Gly Ser His Thr Tyr Val Trp Gln Val Leu
   145                 150                 155                 160
   Lys Glu Asn Gly Pro Met Ala Ser Asp Pro Leu Cys Leu Thr Tyr Ser
                   165                 170                 175
   Tyr Leu Ser His Val Asp Leu Val Lys Asp Leu Asn Ser Gly Leu Ile
               180                 185                 190
   Gly Ala Leu Leu Val Cys Arg Glu Gly Ser Leu Ala Lys Glu Lys Thr
           195                 200                 205
   Gln Thr Leu His Lys Phe Ile Leu Leu Phe Ala Val Phe Asp Glu Gly
       210                 215                 220
   Lys Ser Trp His Ser Glu Thr Lys Asn Ser Leu Met Gln Asp Arg Asp
   225                 230                 235                 240
   Ala Ala Ser Ala Arg Ala Trp Pro Lys Met His Thr Val Asn Gly Tyr
                   245                 250                 255
   Val Asn Arg Ser Leu Pro Gly Leu Ile Gly Cys His Arg Lys Ser Val
               260                 265                 270
   Tyr Trp His Val Ile Gly Met Gly Thr Thr Pro Glu Val His Ser Ile
           275                 280                 285
   Phe Leu Glu Gly His Thr Phe Leu Val Arg Asn His Arg Gln Ala Ser
       290                 295                 300
   Leu Glu Ile Ser Pro Ile Thr Phe Leu Thr Ala Gln Thr Leu Leu Met
   305                 310                 315                 320
   Asp Leu Gly Gln Phe Leu Leu Phe Cys His Ile Ser Ser His Gln His
                   325                 330                 335
   Asp Gly Met Glu Ala Tyr Val Lys Val Asp Ser Cys Pro Glu Glu Pro
               340                 345                 350
   Gln Leu Arg Met Lys Asn Asn Glu Glu Ala Glu Asp Tyr Asp Asp Asp
           355                 360                 365
   Leu Thr Asp Ser Glu Met Asp Val Val Arg Phe Asp Asp Asp Asn Ser
       370                 375                 380
   Pro Ser Phe Ile Gln Ile Arg Ser Val Ala Lys Lys His Pro Lys Thr
   385                 390                 395                 400
   Trp Val His Tyr Ile Ala Ala Glu Glu Glu Asp Trp Asp Tyr Ala Pro
                   405                 410                 415
   Leu Val Leu Ala Pro Asp Asp Arg Ser Tyr Lys Ser Gln Tyr Leu Asn
               420                 425                 430
   Asn Gly Pro Gln Arg Ile Gly Arg Lys Tyr Lys Lys Val Arg Phe Met
           435                 440                 445
   Ala Tyr Thr Asp Glu Thr Phe Lys Thr Arg Glu Ala Ile Gln His Glu
       450                 455                 460
   Ser Gly Ile Leu Gly Pro Leu Leu Tyr Gly Glu Val Gly Asp Thr Leu
   465                 470                 475                 480
   Leu Ile Ile Phe Lys Asn Gln Ala Ser Arg Pro Tyr Asn Ile Tyr Pro
                   485                 490                 495
   His Gly Ile Thr Asp Val Arg Pro Leu Tyr Ser Arg Arg Leu Pro Lys
               500                 505                 510
   Gly Val Lys His Leu Lys Asp Phe Pro Ile Leu Pro Gly Glu Ile Phe
           515                 520                 525
   Lys Tyr Lys Trp Thr Val Thr Val Glu Asp Gly Pro Thr Lys Ser Asp
       530                 535                 540
   Pro Arg Cys Leu Thr Arg Tyr Tyr Ser Ser Phe Val Asn Met Glu Arg
   545                 550                 555                 560
   Asp Leu Ala Ser Gly Leu Ile Gly Pro Leu Leu Ile Cys Tyr Lys Glu
                   565                 570                 575
   Ser Val Asp Gln Arg Gly Asn Gln Ile Met Ser Asp Lys Arg Asn Val
               580                 585                 590
   Ile Leu Phe Ser Val Phe Asp Glu Asn Arg Ser Trp Tyr Leu Thr Glu
           595                 600                 605
   Asn Ile Gln Arg Phe Leu Pro Asn Pro Ala Gly Val Gln Leu Glu Asp
       610                 615                 620
   Pro Glu Phe Gln Ala Ser Asn Ile Met His Ser Ile Asn Gly Tyr Val
   625                 630                 635                 640
   Phe Asp Ser Leu Gln Leu Ser Val Cys Leu His Glu Val Ala Tyr Trp
                   645                 650                 655
   Tyr Ile Leu Ser Ile Gly Ala Gln Thr Asp Phe Leu Ser Val Phe Phe
               660                 665                 670
   Ser Gly Tyr Thr Phe Lys His Lys Met Val Tyr Glu Asp Thr Leu Thr
           675                 680                 685
   Leu Phe Pro Phe Ser Gly Glu Thr Val Phe Met Ser Met Glu Asn Pro
       690                 695                 700
   Gly Leu Trp Ile Leu Gly Cys His Asn Ser Asp Phe Arg Asn Arg Gly
   705                 710                 715                 720
   Met Thr Ala Leu Leu Lys Val Ser Ser Cys Asp Lys Asn Thr Gly Asp
                   725                 730                 735
   Tyr Tyr Glu Asp Ser Tyr Glu Asp Ile Ser Ala Tyr Leu Leu Ser Lys
               740                 745                 750
   Asn Asn Ala Ile Glu Pro Arg Ser Phe Ser Gln Asn Pro Pro Val Leu
           755                 760                 765
   Lys Arg His Gln Arg Glu Ile Thr Arg Thr Thr Leu Gln Ser Asp Gln
       770                 775                 780
   Glu Glu Ile Asp Tyr Asp Asp Thr Ile Ser Val Glu Met Lys Lys Glu
   785                 790                 795                 800
   Asp Phe Asp Ile Tyr Asp Glu Asp Glu Asn Gln Ser Pro Arg Ser Phe
                   805                 810                 815
   Gln Lys Lys Thr Arg His Tyr Phe Ile Ala Ala Val Glu Arg Leu Trp
               820                 825                 830
   Asp Tyr Gly Met Ser Ser Ser Pro His Val Leu Arg Asn Arg Ala Gln
           835                 840                 845
   Ser Gly Ser Val Pro Gln Phe Lys Lys Val Val Phe Gln Glu Phe Thr
       850                 855                 860
   Asp Gly Ser Phe Thr Gln Pro Leu Tyr Arg Gly Glu Leu Asn Glu His
   865                 870                 875                 880
   Leu Gly Leu Leu Gly Pro Tyr Ile Arg Ala Glu Val Glu Asp Asn Ile
                   885                 890                 895
   Met Val Thr Phe Arg Asn Gln Ala Ser Arg Pro Tyr Ser Phe Tyr Ser
               900                 905                 910
   Ser Leu Ile Ser Tyr Glu Glu Asp Gln Arg Gln Gly Ala Glu Pro Arg
           915                 920                 925
   Lys Asn Phe Val Lys Pro Asn Glu Thr Lys Thr Tyr Phe Trp Lys Val
       930                 935                 940
   Gln His His Met Ala Pro Thr Lys Asp Glu Phe Asp Cys Lys Ala Trp
   945                 950                 955                 960
   Ala Tyr Phe Ser Asp Val Asp Leu Glu Lys Asp Val His Ser Gly Leu
                   965                 970                 975
   Ile Gly Pro Leu Leu Val Cys His Thr Asn Thr Leu Asn Pro Ala His
               980                 985                 990
   Gly Arg Gln Val Thr Val Gln Glu  Phe Ala Leu Phe Phe  Thr Ile Phe
           995                 1000                 1005
   Asp Glu  Thr Lys Ser Trp Tyr  Phe Thr Glu Asn Met  Glu Arg Asn
       1010                 1015                 1020
   Cys Arg  Ala Pro Cys Asn Ile  Gln Met Glu Asp Pro  Thr Phe Lys
       1025                 1030                 1035
   Glu Asn  Tyr Arg Phe His Ala  Ile Asn Gly Tyr Ile  Met Asp Thr
       1040                 1045                 1050
   Leu Pro  Gly Leu Val Met Ala  Gln Asp Gln Arg Ile  Arg Trp Tyr
       1055                 1060                 1065
   Leu Leu  Ser Met Gly Ser Asn  Glu Asn Ile His Ser  Ile His Phe
       1070                 1075                 1080
   Ser Gly  His Val Phe Thr Val  Arg Lys Lys Glu Glu  Tyr Lys Met
       1085                 1090                 1095
   Ala Leu  Tyr Asn Leu Tyr Pro  Gly Val Phe Glu Thr  Val Glu Met
       1100                 1105                 1110
   Leu Pro  Ser Lys Ala Gly Ile  Trp Arg Val Glu Cys  Leu Ile Gly
       1115                 1120                 1125
   Glu His  Leu His Ala Gly Met  Ser Thr Leu Phe Leu  Val Tyr Ser
   1130 1135 1140
   Asn Lys Cys Gln Thr Pro Leu Gly Met Ala Ser Gly His Ile Arg
   1145 1150 1155
   Asp Phe Gln Ile Thr Ala Ser Gly Gln Tyr Gly Gln Trp Ala Pro
   1160 1165 1170
   Lys Leu Ala Arg Leu His Tyr Ser Gly Ser Ile Asn Ala Trp Ser
   1175 1180 1185
   Thr Lys Glu Pro Phe Ser Trp Ile Lys Val Asp Leu Leu Ala Pro
   1190 1195 1200
   Met Ile Ile His Gly Ile Lys Thr Gln Gly Ala Arg Gln Lys Phe
   1205 1210 1215
   Ser Ser Leu Tyr Ile Ser Gln Phe Ile Ile Met Tyr Ser Leu Asp
   1220 1225 1230
   Gly Lys Lys Trp Gln Thr Tyr Arg Gly Asn Ser Thr Gly Thr Leu
   1235 1240 1245
   Met Val Phe Phe Gly Asn Val Asp Ser Ser Gly Ile Lys His Asn
   1250 1255 1260
   Ile Phe Asn Pro Pro Ile Ile Ala Arg Tyr Ile Arg Leu His Pro
   1265 1270 1275
   Thr His Tyr Ser Ile Arg Ser Thr Leu Arg Met Glu Leu Met Gly
   1280 1285 1290
   Cys Asp Leu Asn Ser Cys Ser Met Pro Leu Gly Met Glu Ser Lys
   1295 1300 1305
   Ala Ile Ser Asp Ala Gln Ile Thr Ala Ser Ser Tyr Phe Thr Asn
   1310 1315 1320
   Met Phe Ala Thr Trp Ser Pro Ser Lys Ala Arg Leu His Leu Gln
   1325 1330 1335
   Gly Arg Ser Asn Ala Trp Arg Pro Gln Val Asn Asn Pro Lys Glu
   1340 1345 1350
   Trp Leu Gln Val Asp Phe Gln Lys Thr Met Lys Val Thr Gly Val
   1355 1360 1365
   Thr Thr Gln Gly Val Lys Ser Leu Leu Thr Ser Met Tyr Val Lys
   1370 1375 1380
   Glu Phe Leu Ile Ser Ser Ser Gln Asp Gly His Gln Trp Thr Leu
   1385 1390 1395
   Phe Phe Gln Asn Gly Lys Val Lys Val Phe Gln Gly Asn Gln Asp
   1400 1405 1410
   Ser Phe Thr Pro Val Val Asn Ser Leu Asp Pro Pro Leu Leu Thr
   1415 1420 1425
   Arg Tyr Leu Arg Ile His Pro Gln Ser Trp Val His Gln Ile Ala
   1430 1435 1440
   Leu Arg Met Glu Val Leu Gly Cys Glu Ala Gln Asp Leu Tyr
   1445 1450 1455
<210> 4
   <211> 2813
   <212> PRT
   <213> Homo sapiens
<400> 4
   Met Ile Pro Ala Arg Phe Ala Gly Val Leu Leu Ala Leu Ala Leu Ile
   1 5 10 15
   Leu Pro Gly Thr Leu Cys Ala Glu Gly Thr Arg Gly Arg Ser Ser Thr
   20 25 30
   Ala Arg Cys Ser Leu Phe Gly Ser Asp Phe Val Asn Thr Phe Asp Gly
   35 40 45
   Ser Met Tyr Ser Phe Ala Gly Tyr Cys Ser Tyr Leu Leu Ala Gly Gly
   50 55 60
   Cys Gln Lys Arg Ser Phe Ser Ile Ile Gly Asp Phe Gln Asn Gly Lys
   65 70 75 80
   Arg Val Ser Leu Ser Val Tyr Leu Gly Glu Phe Phe Asp Ile His Leu
   85 90 95
   Phe Val Asn Gly Thr Val Thr Gln Gly Asp Gln Arg Val Ser Met Pro
   100 105 110
   Tyr Ala Ser Lys Gly Leu Tyr Leu Glu Thr Glu Ala Gly Tyr Tyr Lys
   115 120 125
   Leu Ser Gly Glu Ala Tyr Gly Phe Val Ala Arg Ile Asp Gly Ser Gly
       130                 135                 140
   Asn Phe Gln Val Leu Leu Ser Asp Arg Tyr Phe Asn Lys Thr Cys Gly
   145                 150                 155                 160
   Leu Cys Gly Asn Phe Asn Ile Phe Ala Glu Asp Asp Phe Met Thr Gln
                   165                 170                 175
   Glu Gly Thr Leu Thr Ser Asp Pro Tyr Asp Phe Ala Asn Ser Trp Ala
               180                 185                 190
   Leu Ser Ser Gly Glu Gln Trp Cys Glu Arg Ala Ser Pro Pro Ser Ser
           195                 200                 205
   Ser Cys Asn Ile Ser Ser Gly Glu Met Gln Lys Gly Leu Trp Glu Gln
       210                 215                 220
   Cys Gln Leu Leu Lys Ser Thr Ser Val Phe Ala Arg Cys His Pro Leu
   225                 230                 235                 240
   Val Asp Pro Glu Pro Phe Val Ala Leu Cys Glu Lys Thr Leu Cys Glu
                   245                 250                 255
   Cys Ala Gly Gly Leu Glu Cys Ala Cys Pro Ala Leu Leu Glu Tyr Ala
               260                 265                 270
   Arg Thr Cys Ala Gln Glu Gly Met Val Leu Tyr Gly Trp Thr Asp His
           275                 280                 285
   Ser Ala Cys Ser Pro Val Cys Pro Ala Gly Met Glu Tyr Arg Gln Cys
       290                 295                 300
   Val Ser Pro Cys Ala Arg Thr Cys Gln Ser Leu His Ile Asn Glu Met
   305                 310                 315                 320
   Cys Gln Glu Arg Cys Val Asp Gly Cys Ser Cys Pro Glu Gly Gln Leu
                   325                 330                 335
   Leu Asp Glu Gly Leu Cys Val Glu Ser Thr Glu Cys Pro Cys Val His
               340                 345                 350
   Ser Gly Lys Arg Tyr Pro Pro Gly Thr Ser Leu Ser Arg Asp Cys Asn
           355                 360                 365
   Thr Cys Ile Cys Arg Asn Ser Gln Trp Ile Cys Ser Asn Glu Glu Cys
       370                 375                 380
   Pro Gly Glu Cys Leu Val Thr Gly Gln Ser His Phe Lys Ser Phe Asp
   385                 390                 395                 400
   Asn Arg Tyr Phe Thr Phe Ser Gly Ile Cys Gln Tyr Leu Leu Ala Arg
                   405                 410                 415
   Asp Cys Gln Asp His Ser Phe Ser Ile Val Ile Glu Thr Val Gln Cys
               420                 425                 430
   Ala Asp Asp Arg Asp Ala Val Cys Thr Arg Ser Val Thr Val Arg Leu
           435                 440                 445
   Pro Gly Leu His Asn Ser Leu Val Lys Leu Lys His Gly Ala Gly Val
       450                 455                 460
   Ala Met Asp Gly Gln Asp Val Gln Leu Pro Leu Leu Lys Gly Asp Leu
   465                 470                 475                 480
   Arg Ile Gln His Thr Val Thr Ala Ser Val Arg Leu Ser Tyr Gly Glu
                   485                 490                 495
   Asp Leu Gln Met Asp Trp Asp Gly Arg Gly Arg Leu Leu Val Lys Leu
               500                 505                 510
   Ser Pro Val Tyr Ala Gly Lys Thr Cys Gly Leu Cys Gly Asn Tyr Asn
           515                 520                 525
   Gly Asn Gln Gly Asp Asp Phe Leu Thr Pro Ser Gly Leu Ala Glu Pro
       530                 535                 540
   Arg Val Glu Asp Phe Gly Asn Ala Trp Lys Leu His Gly Asp Cys Gln
   545                 550                 555                 560
   Asp Leu Gln Lys Gln His Ser Asp Pro Cys Ala Leu Asn Pro Arg Met
                   565                 570                 575
   Thr Arg Phe Ser Glu Glu Ala Cys Ala Val Leu Thr Ser Pro Thr Phe
               580                 585                 590
   Glu Ala Cys His Arg Ala Val Ser Pro Leu Pro Tyr Leu Arg Asn Cys
           595                 600                 605
   Arg Tyr Asp Val Cys Ser Cys Ser Asp Gly Arg Glu Cys Leu Cys Gly
       610                 615                 620
   Ala Leu Ala Ser Tyr Ala Ala Ala Cys Ala Gly Arg Gly Val Arg Val
   625                 630                 635                 640
   Ala Trp Arg Glu Pro Gly Arg Cys Glu Leu Asn Cys Pro Lys Gly Gln
                   645                 650                 655
   Val Tyr Leu Gln Cys Gly Thr Pro Cys Asn Leu Thr Cys Arg Ser Leu
               660                 665                 670
   Ser Tyr Pro Asp Glu Glu Cys Asn Glu Ala Cys Leu Glu Gly Cys Phe
           675                 680                 685
   Cys Pro Pro Gly Leu Tyr Met Asp Glu Arg Gly Asp Cys Val Pro Lys
       690                 695                 700
   Ala Gln Cys Pro Cys Tyr Tyr Asp Gly Glu Ile Phe Gln Pro Glu Asp
   705                 710                 715                 720
   Ile Phe Ser Asp His His Thr Met Cys Tyr Cys Glu Asp Gly Phe Met
                   725                 730                 735
   His Cys Thr Met Ser Gly Val Pro Gly Ser Leu Leu Pro Asp Ala Val
               740                 745                 750
   Leu Ser Ser Pro Leu Ser His Arg Ser Lys Arg Ser Leu Ser Cys Arg
           755                 760                 765
   Pro Pro Met Val Lys Leu Val Cys Pro Ala Asp Asn Leu Arg Ala Glu
       770                 775                 780
   Gly Leu Glu Cys Thr Lys Thr Cys Gln Asn Tyr Asp Leu Glu Cys Met
   785                 790                 795                 800
   Ser Met Gly Cys Val Ser Gly Cys Leu Cys Pro Pro Gly Met Val Arg
                   805                 810                 815
   His Glu Asn Arg Cys Val Ala Leu Glu Arg Cys Pro Cys Phe His Gln
               820                 825                 830
   Gly Lys Glu Tyr Ala Pro Gly Glu Thr Val Lys Ile Gly Cys Asn Thr
           835                 840                 845
   Cys Val Cys Arg Asp Arg Lys Trp Asn Cys Thr Asp His Val Cys Asp
       850                 855                 860
   Ala Thr Cys Ser Thr Ile Gly Met Ala His Tyr Leu Thr Phe Asp Gly
   865                 870                 875                 880
   Leu Lys Tyr Leu Phe Pro Gly Glu Cys Gln Tyr Val Leu Val Gln Asp
   885 890 895
   Tyr Cys Gly Ser Asn Pro Gly Thr Phe Arg Ile Leu Val Gly Asn Lys
   900 905 910
   Gly Cys Ser His Pro Ser Val Lys Cys Lys Lys Arg Val Thr Ile Leu
   915 920 925
   Val Glu Gly Gly Glu Ile Glu Leu Phe Asp Gly Glu Val Asn Val Lys
   930 935 940
   Arg Pro Met Lys Asp Glu Thr His Phe Glu Val Val Glu Ser Gly Arg
   945 950 955 960
   Tyr Ile Ile Leu Leu Leu Gly Lys Ala Leu Ser Val Val Trp Asp Arg
   965 970 975
   His Leu Ser Ile Ser Val Val Leu Lys Gln Thr Tyr Gln Glu Lys Val
   980 985 990
   Cys Gly Leu Cys Gly Asn Phe Asp Gly Ile Gln Asn Asn Asp Leu Thr
   995 1000 1005
   Ser Ser Asn Leu Gln Val Glu Glu Asp Pro Val Asp Phe Gly Asn
   1010 1015 1020
   Ser Trp Lys Val Ser Ser Gln Cys Ala Asp Thr Arg Lys Val Pro
   1025 1030 1035
   Leu Asp Ser Ser Pro Ala Thr Cys His Asn Asn Ile Met Lys Gln
   1040 1045 1050
   Thr Met Val Asp Ser Ser Cys Arg Ile Leu Thr Ser Asp Val Phe
   1055 1060 1065
   Gln Asp Cys Asn Lys Leu Val Asp Pro Glu Pro Tyr Leu Asp Val
   1070 1075 1080
   Cys Ile Tyr Asp Thr Cys Ser Cys Glu Ser Ile Gly Asp Cys Ala
   1085 1090 1095
   Cys Phe Cys Asp Thr Ile Ala Ala Tyr Ala His Val Cys Ala Gln
   1100 1105 1110
   His Gly Lys Val Val Thr Trp Arg Thr Ala Thr Leu Cys Pro Gln
   1115 1120 1125
   Ser Cys Glu Glu Arg Asn Leu Arg Glu Asn Gly Tyr Glu Cys Glu
   1130 1135 1140
   Trp Arg Tyr Asn Ser Cys Ala Pro Ala Cys Gln Val Thr Cys Gln
   1145 1150 1155
   His Pro Glu Pro Leu Ala Cys Pro Val Gln Cys Val Glu Gly Cys
   1160 1165 1170
   His Ala His Cys Pro Pro Gly Lys Ile Leu Asp Glu Leu Leu Gln
   1175 1180 1185
   Thr Cys Val Asp Pro Glu Asp Cys Pro Val Cys Glu Val Ala Gly
   1190 1195 1200
   Arg Arg Phe Ala Ser Gly Lys Lys Val Thr Leu Asn Pro Ser Asp
   1205 1210 1215
   Pro Glu His Cys Gln Ile Cys His Cys Asp Val Val Asn Leu Thr
   1220 1225 1230
   Cys Glu Ala Cys Gln Glu Pro Gly Gly Leu Val Val Pro Pro Thr
   1235 1240 1245
   Asp Ala Pro Val Ser Pro Thr Thr Leu Tyr Val Glu Asp Ile Ser
   1250 1255 1260
   Glu Pro Pro Leu His Asp Phe Tyr Cys Ser Arg Leu Leu Asp Leu
   1265 1270 1275
   Val Phe Leu Leu Asp Gly Ser Ser Arg Leu Ser Glu Ala Glu Phe
   1280 1285 1290
   Glu Val Leu Lys Ala Phe Val Val Asp Met Met Glu Arg Leu Arg
   1295 1300 1305
   Ile Ser Gln Lys Trp Val Arg Val Ala Val Val Glu Tyr His Asp
   1310 1315 1320
   Gly Ser His Ala Tyr Ile Gly Leu Lys Asp Arg Lys Arg Pro Ser
   1325 1330 1335
   Glu Leu Arg Arg Ile Ala Ser Gln Val Lys Tyr Ala Gly Ser Gln
   1340 1345 1350
   Val Ala Ser Thr Ser Glu Val Leu Lys Tyr Thr Leu Phe Gln Ile
   1355 1360 1365
   Phe Ser Lys Ile Asp Arg Pro Glu Ala Ser Arg Ile Thr Leu Leu
   1370 1375 1380
   Leu Met Ala Ser Gln Glu Pro Gln Arg Met Ser Arg Asn Phe Val
   1385 1390 1395
   Arg Tyr Val Gln Gly Leu Lys Lys Lys Lys Val Ile Val Ile Pro
   1400 1405 1410
   Val Gly Ile Gly Pro His Ala Asn Leu Lys Gln Ile Arg Leu Ile
   1415 1420 1425
   Glu Lys Gln Ala Pro Glu Asn Lys Ala Phe Val Leu Ser Ser Val
   1430 1435 1440
   Asp Glu Leu Glu Gln Gln Arg Asp Glu Ile Val Ser Tyr Leu Cys
   1445 1450 1455
   Asp Leu Ala Pro Glu Ala Pro Pro Pro Thr Leu Pro Pro Asp Met
   1460 1465 1470
   Ala Gln Val Thr Val Gly Pro Gly Leu Leu Gly Val Ser Thr Leu
   1475 1480 1485
   Gly Pro Lys Arg Asn Ser Met Val Leu Asp Val Ala Phe Val Leu
   1490 1495 1500
   Glu Gly Ser Asp Lys Ile Gly Glu Ala Asp Phe Asn Arg Ser Lys
   1505 1510 1515
   Glu Phe Met Glu Glu Val Ile Gln Arg Met Asp Val Gly Gln Asp
   1520 1525 1530
   Ser Ile His Val Thr Val Leu Gln Tyr Ser Tyr Met Val Thr Val
   1535 1540 1545
   Glu Tyr Pro Phe Ser Glu Ala Gln Ser Lys Gly Asp Ile Leu Gln
   1550 1555 1560
   Arg Val Arg Glu Ile Arg Tyr Gln Gly Gly Asn Arg Thr Asn Thr
   1565 1570 1575
   Gly Leu Ala Leu Arg Tyr Leu Ser Asp His Ser Phe Leu Val Ser
   1580 1585 1590
   Gln Gly Asp Arg Glu Gln Ala Pro Asn Leu Val Tyr Met Val Thr
   1595 1600 1605
   Gly Asn  Pro Ala Ser Asp Glu  Ile Lys Arg Leu Pro  Gly Asp Ile
       1610                 1615                 1620
   Gln Val  Val Pro Ile Gly Val  Gly Pro Asn Ala Asn  Val Gln Glu
       1625                 1630                 1635
   Leu Glu  Arg Ile Gly Trp Pro  Asn Ala Pro Ile Leu  Ile Gln Asp
       1640                 1645                 1650
   Phe Glu  Thr Leu Pro Arg Glu  Ala Pro Asp Leu Val  Leu Gln Arg
       1655                 1660                 1665
   Cys Cys  Ser Gly Glu Gly Leu  Gln Ile Pro Thr Leu  Ser Pro Ala
       1670                 1675                 1680
   Pro Asp  Cys Ser Gln Pro Leu  Asp Val Ile Leu Leu  Leu Asp Gly
       1685                 1690                 1695
   Ser Ser  Ser Phe Pro Ala Ser  Tyr Phe Asp Glu Met  Lys Ser Phe
       1700                 1705                 1710
   Ala Lys  Ala Phe Ile Ser Lys  Ala Asn Ile Gly Pro  Arg Leu Thr
       1715                 1720                 1725
   Gln Val  Ser Val Leu Gln Tyr  Gly Ser Ile Thr Thr  Ile Asp Val
       1730                 1735                 1740
   Pro Trp  Asn Val Val Pro Glu  Lys Ala His Leu Leu  Ser Leu Val
       1745                 1750                 1755
   Asp Val  Met Gln Arg Glu Gly  Gly Pro Ser Gln Ile  Gly Asp Ala
       1760                 1765                 1770
   Leu Gly  Phe Ala Val Arg Tyr  Leu Thr Ser Glu Met  His Gly Ala
       1775                 1780                 1785
   Arg Pro  Gly Ala Ser Lys Ala  Val Val Ile Leu Val  Thr Asp Val
       1790                 1795                 1800
   Ser Val  Asp Ser Val Asp Ala  Ala Ala Asp Ala Ala  Arg Ser Asn
       1805                 1810                 1815
   Arg Val  Thr Val Phe Pro Ile  Gly Ile Gly Asp Arg  Tyr Asp Ala
       1820                 1825                 1830
   Ala Gln  Leu Arg Ile Leu Ala  Gly Pro Ala Gly Asp  Ser Asn Val
       1835                1840                1845
   Val Lys  Leu Gln Arg Ile Glu  Asp Leu Pro Thr Met  Val Thr Leu
       1850                1855                1860
   Gly Asn  Ser Phe Leu His Lys  Leu Cys Ser Gly Phe  Val Arg Ile
       1865                1870                1875
   Cys Met  Asp Glu Asp Gly Asn  Glu Lys Arg Pro Gly  Asp Val Trp
       1880                1885                1890
   Thr Leu  Pro Asp Gln Cys His  Thr Val Thr Cys Gln  Pro Asp Gly
       1895                1900                1905
   Gln Thr  Leu Leu Lys Ser His  Arg Val Asn Cys Asp  Arg Gly Leu
       1910                1915                1920
   Arg Pro  Ser Cys Pro Asn Ser  Gln Ser Pro Val Lys  Val Glu Glu
       1925                1930                1935
   Thr Cys  Gly Cys Arg Trp Thr  Cys Pro Cys Val Cys  Thr Gly Ser
       1940                1945                1950
   Ser Thr  Arg His Ile Val Thr  Phe Asp Gly Gln Asn  Phe Lys Leu
       1955                1960                1965
   Thr Gly  Ser Cys Ser Tyr Val  Leu Phe Gln Asn Lys  Glu Gln Asp
       1970                1975                1980
   Leu Glu  Val Ile Leu His Asn  Gly Ala Cys Ser Pro  Gly Ala Arg
       1985                1990                1995
   Gln Gly  Cys Met Lys Ser Ile  Glu Val Lys His Ser  Ala Leu Ser
       2000                2005                2010
   Val Glu  Leu His Ser Asp Met  Glu Val Thr Val Asn  Gly Arg Leu
       2015                2020                2025
   Val Ser  Val Pro Tyr Val Gly  Gly Asn Met Glu Val  Asn Val Tyr
       2030                2035                2040
   Gly Ala  Ile Met His Glu Val  Arg Phe Asn His Leu  Gly His Ile
       2045                2050                2055
   Phe Thr  Phe Thr Pro Gln Asn  Asn Glu Phe Gln Leu  Gln Leu Ser
       2060                2065                2070
   Pro Lys Thr Phe Ala Ser Lys Thr Tyr Gly Leu Cys Gly Ile Cys
   2075 2080 2085
   Asp Glu Asn Gly Ala Asn Asp Phe Met Leu Arg Asp Gly Thr Val
   2090 2095 2100
   Thr Thr Asp Trp Lys Thr Leu Val Gln Glu Trp Thr Val Gln Arg
   2105 2110 2115
   Pro Gly Gln Thr Cys Gln Pro Ile Leu Glu Glu Gln Cys Leu Val
   2120 2125 2130
   Pro Asp Ser Ser His Cys Gln Val Leu Leu Leu Pro Leu Phe Ala
   2135 2140 2145
   Glu Cys His Lys Val Leu Ala Pro Ala Thr Phe Tyr Ala Ile Cys
   2150 2155 2160
   Gln Gln Asp Ser Cys His Gln Glu Gln Val Cys Glu Val Ile Ala
   2165 2170 2175
   Ser Tyr Ala His Leu Cys Arg Thr Asn Gly Val Cys Val Asp Trp
   2180 2185 2190
   Arg Thr Pro Asp Phe Cys Ala Met Ser Cys Pro Pro Ser Leu Val
   2195 2200 2205
   Tyr Asn His Cys Glu His Gly Cys Pro Arg His Cys Asp Gly Asn
   2210 2215 2220
   Val Ser Ser Cys Gly Asp His Pro Ser Glu Gly Cys Phe Cys Pro
   2225 2230 2235
   Pro Asp Lys Val Met Leu Glu Gly Ser Cys Val Pro Glu Glu Ala
   2240 2245 2250
   Cys Thr Gln Cys Ile Gly Glu Asp Gly Val Gln His Gln Phe Leu
   2255 2260 2265
   Glu Ala Trp Val Pro Asp His Gln Pro Cys Gln Ile Cys Thr Cys
   2270 2275 2280
   Leu Ser Gly Arg Lys Val Asn Cys Thr Thr Gln Pro Cys Pro Thr
   2285 2290 2295
   Ala Lys Ala Pro Thr Cys Gly Leu Cys Glu Val Ala Arg Leu Arg
   2300 2305 2310
   Gln Asn  Ala Asp Gln Cys Cys  Pro Glu Tyr Glu Cys  Val Cys Asp
       2315                 2320                 2325
   Pro Val  Ser Cys Asp Leu Pro  Pro Val Pro His Cys  Glu Arg Gly
       2330                 2335                 2340
   Leu Gln  Pro Thr Leu Thr Asn  Pro Gly Glu Cys Arg  Pro Asn Phe
       2345                 2350                 2355
   Thr Cys  Ala Cys Arg Lys Glu  Glu Cys Lys Arg Val  Ser Pro Pro
       2360                 2365                 2370
   Ser Cys  Pro Pro His Arg Leu  Pro Thr Leu Arg Lys  Thr Gln Cys
       2375                 2380                 2385
   Cys Asp  Glu Tyr Glu Cys Ala  Cys Asn Cys Val Asn  Ser Thr Val
       2390                 2395                 2400
   Ser Cys  Pro Leu Gly Tyr Leu  Ala Ser Thr Ala Thr  Asn Asp Cys
       2405                 2410                 2415
   Gly Cys  Thr Thr Thr Thr Cys  Leu Pro Asp Lys Val  Cys Val His
       2420                 2425                 2430
   Arg Ser  Thr Ile Tyr Pro Val  Gly Gln Phe Trp Glu  Glu Gly Cys
       2435                 2440                 2445
   Asp Val  Cys Thr Cys Thr Asp  Met Glu Asp Ala Val  Met Gly Leu
       2450                 2455                 2460
   Arg Val  Ala Gln Cys Ser Gln  Lys Pro Cys Glu Asp  Ser Cys Arg
       2465                 2470                 2475
   Ser Gly  Phe Thr Tyr Val Leu  His Glu Gly Glu Cys  Cys Gly Arg
       2480                 2485                 2490
   Cys Leu  Pro Ser Ala Cys Glu  Val Val Thr Gly Ser  Pro Arg Gly
       2495                 2500                 2505
   Asp Ser  Gln Ser Ser Trp Lys  Ser Val Gly Ser Gln  Trp Ala Ser
       2510                 2515                 2520
   Pro Glu  Asn Pro Cys Leu Ile  Asn Glu Cys Val Arg  Val Lys Glu
       2525                 2530                 2535
   Glu Val  Phe Ile Gln Gln Arg  Asn Val Ser Cys Pro  Gln Leu Glu
       2540                 2545                 2550
   Val Pro Val Cys Pro Ser Gly Phe Gln Leu Ser Cys Lys Thr Ser
   2555 2560 2565
   Ala Cys Cys Pro Ser Cys Arg Cys Glu Arg Met Glu Ala Cys Met
   2570 2575 2580
   Leu Asn Gly Thr Val Ile Gly Pro Gly Lys Thr Val Met Ile Asp
   2585 2590 2595
   Val Cys Thr Thr Cys Arg Cys Met Val Gln Val Gly Val Ile Ser
   2600 2605 2610
   Gly Phe Lys Leu Glu Cys Arg Lys Thr Thr Cys Asn Pro Cys Pro
   2615 2620 2625
   Leu Gly Tyr Lys Glu Glu Asn Asn Thr Gly Glu Cys Cys Gly Arg
   2630 2635 2640
   Cys Leu Pro Thr Ala Cys Thr Ile Gln Leu Arg Gly Gly Gln Ile
   2645 2650 2655
   Met Thr Leu Lys Arg Asp Glu Thr Leu Gln Asp Gly Cys Asp Thr
   2660 2665 2670
   His Phe Cys Lys Val Asn Glu Arg Gly Glu Tyr Phe Trp Glu Lys
   2675 2680 2685
   Arg Val Thr Gly Cys Pro Pro Phe Asp Glu His Lys Cys Leu Ala
   2690 2695 2700
   Glu Gly Gly Lys Ile Met Lys Ile Pro Gly Thr Cys Cys Asp Thr
   2705 2710 2715
   Cys Glu Glu Pro Glu Cys Asn Asp Ile Thr Ala Arg Leu Gln Tyr
   2720 2725 2730
   Val Lys Val Gly Ser Cys Lys Ser Glu Val Glu Val Asp Ile His
   2735 2740 2745
   Tyr Cys Gln Gly Lys Cys Ala Ser Lys Ala Met Tyr Ser Ile Asp
   2750 2755 2760
   Ile Asn Asp Val Gln Asp Gln Cys Ser Cys Cys Ser Pro Thr Arg
   2765 2770 2775
   Thr Glu Pro Met Gln Val Ala Leu His Cys Thr Asn Gly Ser Val
                   2780                2785                2790
   Val Tyr  His Glu Val Leu Asn  Ala Met Glu Cys Lys  Cys Ser Pro
       2795                2800                2805
   Arg Lys  Cys Ser Lys
       2810

## Description

### Field of Invention

The present invention relates to the functional expression of recombinant factor VIII (rFVIII) in cells and in particular to its stable expression and excretion from cells in *in vitro* and/or *in vivo* applications.

### Background to Invention

Factor VIII (FVIII) is a large, complex glycoprotein. It is synthesized as a 330-kDa protein with the domain structure A1-A2-B-A3-C1-C2 where both A and C domains have internal sequence homology and approximately 40% sequence identity to the A and C domains of factor V. The B-domain, which constitutes 38% of the total sequence, shares no sequence identity with other known proteins, including the B-domain of factor V. It is, however extensively glycosylated and contains 19 of the 26 asparagine (N)-linked glycosylation sites on the whole molecule.

Much insight into the biosynthesis of FVIII has come from the analysis of expression in heterologous cell types with the purpose of producing rFVIII for therapeutic purposes. In addition, characterisation of the molecular defects responsible for the autosomal recessive bleeding disorder associated with combined deficiency of factors V and VIII revealed the importance of intracellular trafficking in the biosynthesis of FVIII (1,2).

Early studies of rFVIII demonstrated that the B domain could be deleted without loss of FVIII procoagulant activity (reviewed in 3). Expression cassettes encoding B domain-deleted rFVIII resulted in significantly higher levels of mRNA over equivalent full-length rFVIII expression cassettes, resulting in higher levels of rFVIII protein synthesis. However, the levels of rFVIII secreted into the media did not increase proportionately, suggesting intracellular interactions were limiting efficient secretion (4). rFVIII is co-translationally translocated into the lumen of the ER where it folds and assembles into tertiary structures. These reactions are facilitated by enzymes and molecular chaperones that interact with folding intermediates of rFVIII.

In vivo, immediately after its secretion into the circulation FVIII interacts to form a tight non-covalent complex that protects it from premature clearance or inactivation by proteolysis. FVIII binds to von willebrand factor (VWF) through two sites in the A3 and C2 domains. *In vitro* it has been shown that the addition of VWF to the culture media or co-expression through transfection with a VWF expression cassette of cells expressing rFVIII leads to increased accumulation of rFVIII in the media by increasing the stability of the rFVIII in the culture media (6). It has been proposed that VWF promotes the dissociation of rFVIII from the cell surface and may also promote the assembly of the two chain hetero-dimer of the heavy and light chains of rFVIII (14, 15), however it is only thought to from a non-covalent complex with FVIII once outside the cell.

Deletion, of the entire B-domain which led to a 17-fold increase in mRNA and primary translation product only resulted in a 30% increase in the levels of secreted protein, suggesting that the rate of ER-Golgi transport was reduced and that levels of FVIII mRNA were not limiting expression (4). The introduction of multiple N-linked glycosylation sites known to be important in ER-Golgi transport of FVIII increased levels of secreted FVIII, suggesting that the rate of ER-Golgi transport may be a rate limiting step (7). However, a significant amount of FVIII within the ER never transits to the Golgi compartment due to a failure to fold correctly and misfolded FVIII accumulation in the ER can result in oxidative damage and apoptosis (5), suggesting that FVIII folding is the rate-limiting step in FVIII expression.

In a recent study designed to investigate the effect of FVIII expression cassettes with various B-domain constructs the effect of codon optimising these sequences for expression in *Homo sapiens* was also investigated (8). Human embryonic kidney cells (HEK293T) cells were transiently transduced and expression of FVIII was assessed 72h latter. Surprisingly no significant effect of incorporation of the B domain regions was observed, however a 7 to 30-fold increase in functional FVIII expression from codon optimised sequences was observed. Although protein secondary structure is determined primarily by the amino acid sequence, protein folding within the cell is affected by a range of factors: these include interaction with other proteins (chaperones) and ligands, translocation through the ER membrane and redox conditions. The rate of translation can also affect protein folding and it has been suggested that codon usage may be a mechanism to regulate translation speed and thus allow stepwise folding of individual protein domains (9,10). FVIII is a complex multi-domain protein in which non-sequential segments of the nascent polypeptide chain may interact in the three dimensional fold. Ribosome stalling at 'rare' codons may therefore lead to alternative folding pathways generating altered conformations and potentially misfolded protein. A potential explanation for the observed effect of codon optimised sequences may be that they allow efficient translation and transport across the ER membrane allowing the nascent FVIII polypeptide chain to fold correctly leading to the increased levels of secreted FVIII observed in vitro and in vivo.
Spencer et al (16) teaches the use of a Lentiviral vector platform to express a bioengineered recombinant Factor VIII at very high levels. However this is achieved by creating a chimeric FVIII sequence which still includes 12% porcine sequence and a such the FVIII is not non truly a native or human sequence. This may cause regulatory concerns with regards its use so this may not be desirable, particularly in a human context. Kaufman et al., Mol. Cell. Biol. vol.9, No.3. p.1233-1242 (March 1989) and Chen et al., Thrombosis Research vol. 95, p. 105-115 (1999) describe co-expression of recombinant FVIII and VWF in mammalian cell lines. However, they do not address the problem of improving long-term FVIII production nor do they comment on codon-optimisation.

### Summary of the Invention

The present invention is based on studies performed by the inventors into B domain deleted (BDD) rFVIII expression in cells and in particular codon optimised rFVIII.

The invention is based on work carried out by the present inventors towards providing high-yield production of versions of mammalian, especially human FVIII tailored for animal and human trials and therapeutic applications, which is based on the use of codon optimised chemically synthesised genes that are transfected into, for example mammalian cells either in vitro or in vivo.

The inventors have observed that mammalian cells transfected with hamster codon optimised B domain deleted recombinant FVIII (BDDFVIII coop) did not remain viable over a period of time and that the BDDFVIII coop could not therefore be obtained stably in useful quantities. However by co-expressing BDDFVIII coop with similarly hamster codon optimised VWF, stably transfected cells could be obtained, which remain viable for a significant period of time and from which a high level of expression of BDDFVIII coop could be observed. Kaufman (6) suggests that co-expression of VWF with rFVIII leads to extra-cellular stability of rFVIII, but there was no suggestion that co-expression of VWF with BDDFVIII coop might help maintain cell viability, as well as functional viability of BDDFVIII coop.

In a first aspect there is provided a mammalian cell comprising an exogenously introduced nucleic acid which is capable of recombinantly expressing FVIII wherein the nucleic acid, or portion thereof which encodes said FVIII has been codon optimised for expression by the mammalian cell; and wherein the cell further recombinantly co-expresses von Willebrand Factor (VWF).

In accordance with the present invention the nucleic acid which expresses VWF is introduced into the mammalian cell prior to the nucleic acid which is capable of recombinantly expressing FVIII.

In a preferred embodiment the VWF polynucleotide sequence may also be codon optimized in a similar manner to the FVIII sequence. Thus, although the remainder of the specification refers to codon optimisation and expression of FVIII in particular, this is not to be construed as limiting and may extend in a similar manner to VWF.

The mammalian FVIII referred to is preferably human FVIII, but may be FVIII from another primate or other mammal, such as that from mouse, rat, hamster, rabbit, hamster, dog, horse, cow, pig, sheep, camel, cat, guinea pig, or the like. The FVIII may be a modified version as known in the art, for example a B domain deleted version of BDD FVIII.

The mammalian host cell may be any cell suitable for expression of codon optimised FVIII and may for example be of human, mouse, hamster, rat, monkey, rabbit or dog origin. Naturally the FVIII or modified version thereof will be codon optimised as known in the art and described further below so as to be suitably codon optimised for expression in the chosen cell. For example if the cell is of hamster origin the FVIII nucleic acid coding sequence will be codon optimised for hamster expression. Cell lines which may be suitable for expression include HEK 293, CHO, 3T3, BHK, MICK, Vero, Junket, Hela, as well as derivatives of such cells known in the art.

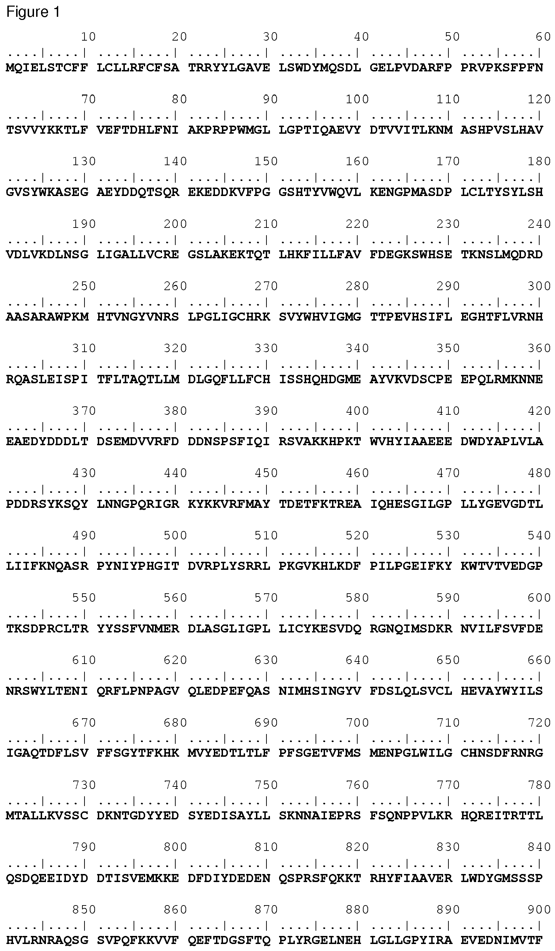

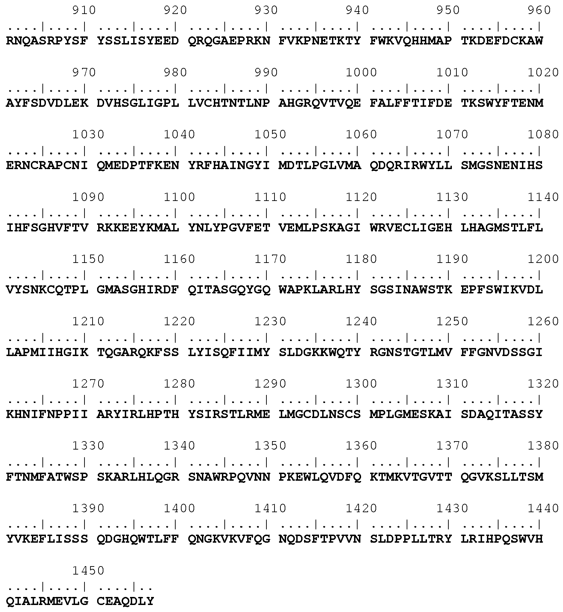

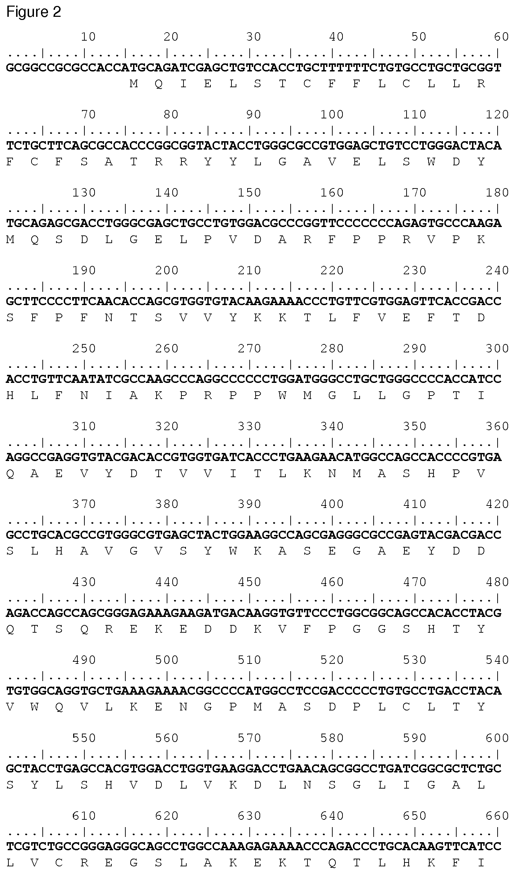

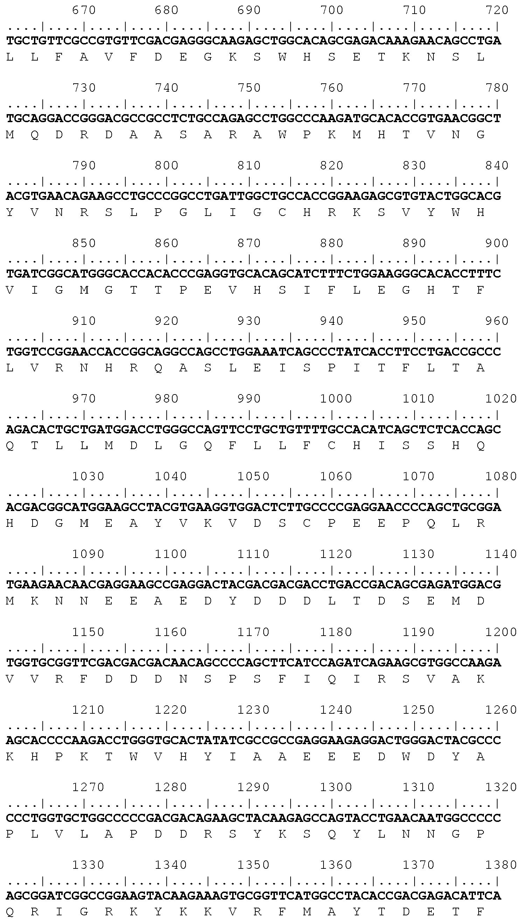

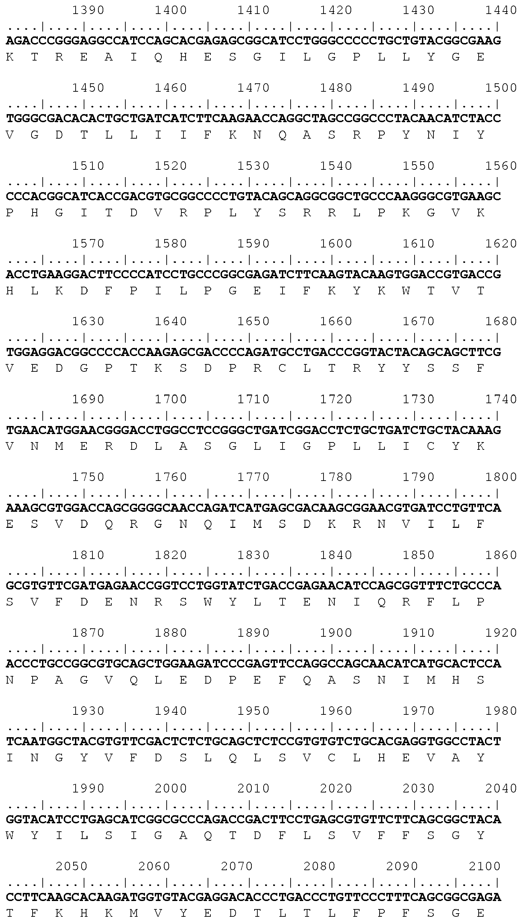

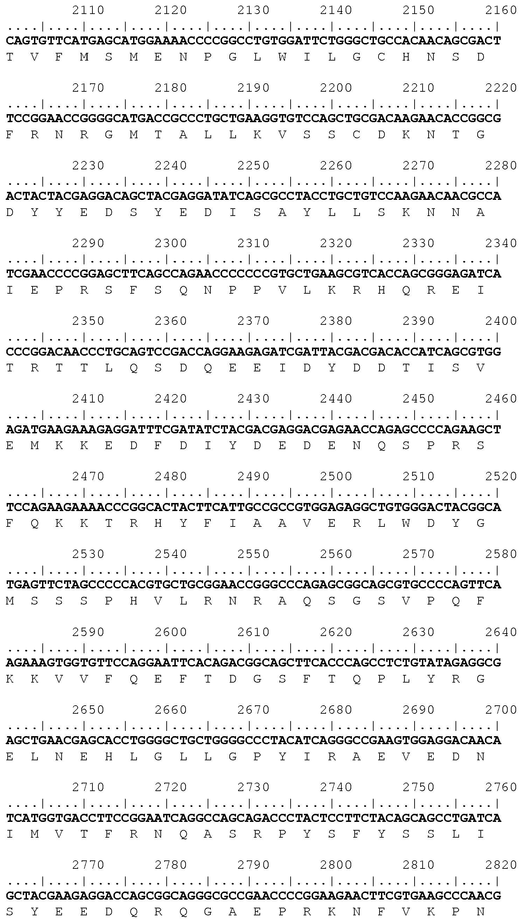

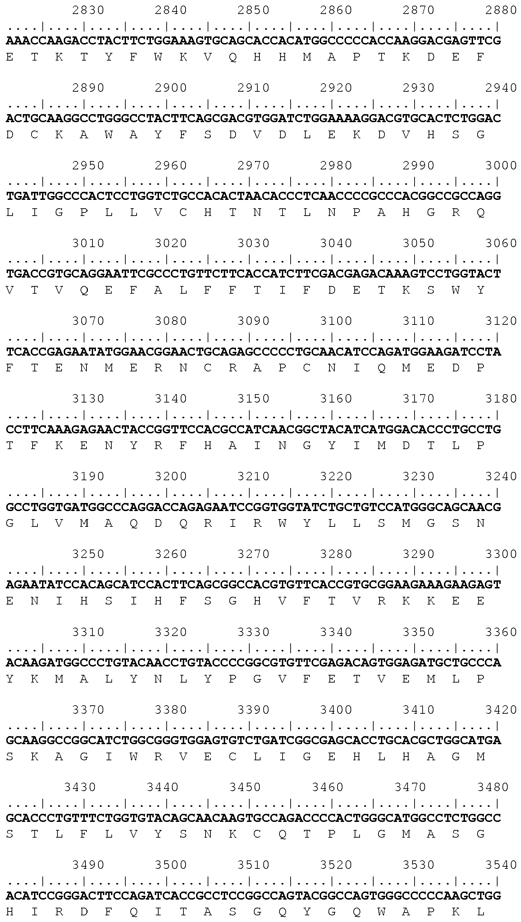

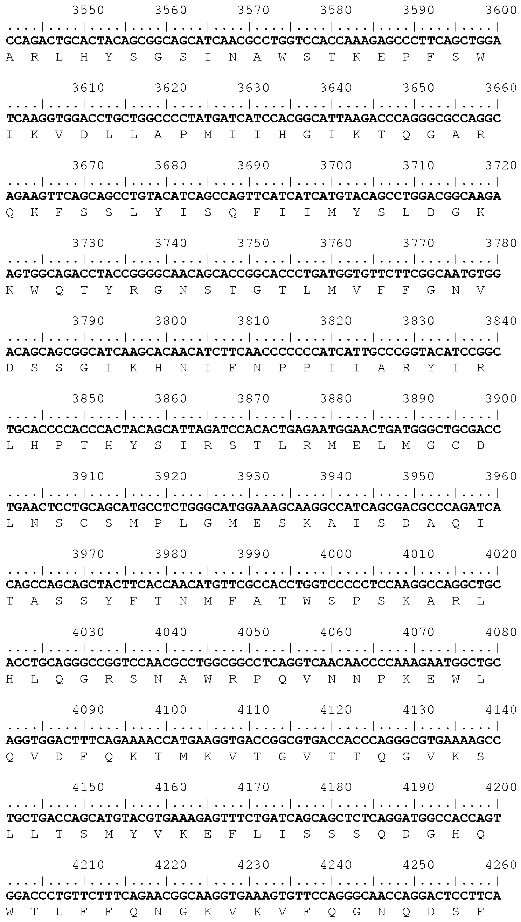

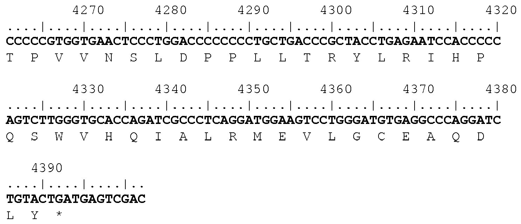

Although the nucleic acid may be codon optimised for expression in a particular mammalian cell, the amino acid sequence may conform to FVIII from the same or a different species. In a preferred embodiment the FVIII amino acid sequence corresponds to a human FVIII sequence or a fragment thereof. Suitable human or other animal FVIII fragments include in a preferred embodiment, a FVIII sequence from which the B domain has been deleted, and optionally replaced with a short B-domain spacer rich in asparagine-linked oligosaccharides. A preferred FVIII B-domain deleted amino acid sequence is shown in Figure 1. Such a sequence when codon optimised for expression in hamster, such as CHO or BHK cells is shown in Figure 2.

In certain embodiments, the present invention utilises an isolated polynucleotide comprising a nucleic acid fragment which encodes a polypeptide at least 90%, 91%, 92%, 93%, 94%, 95%, 96%, 97%, 98%, or 99% identical to the sequence shown in Figure 2, and where the nucleic acid fragment encodes or is a variant of a codon optimised FVIII, such as human FVIII represented by Figure 1. The human codon optimised coding region can be optimised by any of the methods described herein.

Typically the nucleic acid is engineered within an expression vector, which is capable of expressing said recombinant FVIII under suitable conditions.

Conveniently, the codon optimised nucleic acid sequence can initially be chemically synthesised rather than cloned and mutagenised in order to generate the necessary codon optimisation. In accordance with the present invention it is possible to produce large quantities of recombinant mammalian FVIII and its variants hitherto not possible using the previously described techniques. Typically the methods of the present invention may produce protein yields of at least 0.5 mg of recombinant FVIII (or its variants) per liter of culture medium, such as at least 1 mg, 5mg, 10 mg, 50 mg, 100 mg, 200 mg or 500 mg per litre of culture medium. It will therefore be appreciated that it is possible following the methods of the present invention, when using industrial-scale fermentors, to produce hundreds of milligrams or grams, or even kilogram-quantities of recombinant FVIII and variants thereof. It will also be further appreciated that it is possible following the methods of the present invention to achieve protein yields comparable with or greater than the productivity of industrial production cell lines for FVIII (i.e.>0.05 pg/cell/day. This may or may not require the use of gene amplification techniques such as DHFR/methotrexate).

In a further aspect the present invention provides a cell culture comprising a mammalian cell as described herein according to any preceding claim, which is capable of expressing the FVIII in an amount of ≥ 0.1pg/cell/day, typically ≥ 0.25 pg/cell/day.

The cell culture may be considered to be stable in that it comprises one or more of the following features:
1) the cell culture comprises at least 5 x 10⁶, 1 x 10⁷ or 1 x 10⁸ cells per ml;
2) a cell viability of at least 80%, 90%, 95% or 97%; and
3) cell viability is maintained for at least 2 weeks, 3 weeks or even 4 weeks.

In a further aspect there is provided a method of preparing recombinant mammalian FVIII or a B domain deleted version of FVIII, comprising providing a cell, transfecting the cell with a vector capable of recombinantly expressing VWF and thereafter further transfecting cells transfected with VWF with a vector capable of recombinantly expressing FVIII or a B domain deleted version of FVIII, wherein the transfections are carried out with vectors which comprise codon optimised nucleic acid constructs capable of expressing FVIII or a B domain deleted version of FVIII and VWF in said cell. Advantageously, the present method may not require, in some embodiments, the use of gene amplification techniques such as DHFR/methotrexate amplification methods known in the art (see for example, Kaufman (17). The inventors have observed that methotrexate at levels of up to 0.5µM mg be used to increase productivity of recombinant FVIII, but higher levels have a deleterious effect, which was unexpected.

The above process may further comprise purifying said proteins or complex from the cell and/or culture medium in which the cell is grown. Purification may typically involve the use of chromatographic methodologies, such as fast-protein liquid chromatographic or high-performance (pressure) liquid chromatographic techniques known in the art. In a subsequent purification step, crude material may, for example, be loaded onto a sepharose or agarose based affinity chromatography column, containing an affinity ligand for VWF or FVIII such as a monoclonal antibody or a peptide ligand. In a preferred embodiment an antibody to VWF (CaptureSelect VWF; Life Technologies) or FVIII (FVIIISelect; GE Healthcare) is employed in order to bind to said FVIII and/or VWF, and eluted by application of a salt gradient (*e.g*.1-1.5 M NaCl or CaCl₂) over multiple column volumes; in a further step, FVIII containing fractions from the previous step(s) may be loaded onto, for example, anion exchange and/or HIC columns. The order of such purification steps may be altered to increase yield and/or purity of the rFVIII or variant thereof.

Rather than conventional gene cloning and expression, the present invention is based on an initial chemical synthesis of the codon optimised DNA molecules encoding said FVIII (and variants thereof), using gene design and synthesis techniques in the art (e.g.Gene composer: database software for protein construct design, codon engineering, and gene synthesis, see Lorimer (18) for example. In this manner, the codon optimised nucleic acid is synthesised *de novo* prior to cloning into a suitable expression vector. Conventional site-directed mutagenesis techniques known in the art to carry out codon optimisation of the FVIII gene would be unfeasibly time-consuming, if not impossible due to the high risk of introducing additional mutational variations during the requisite repeated rounds of site-directed mutagenesis. However, site-directed mutagenesis may be used following cloning of the synthetic codon optimised FVIII, in order to accomplish one or a combination of site-specific mutations in the product.

Codon optimisation is carried out in order to enhance the expression levels of the mammalian FVIII and its variants in the desired host organism or cell type, such as hamster or hamster cells. Said optimisation involves one or more of the following: adapting codon bias to match that of the chosen host organism or indeed cell type; avoiding regions of high (>80%) or low (<30%) GC content; minimising any potential internal TATA boxes, chi-sites and ribosome-entry sites; minimising AT-rich or GC-rich stretches of sequence, avoiding repeat sequence and RNA secondary structures, minimising any (cryptic) splice-donor and/or splice-acceptor sites; and ensuring any desired restriction endonuclease sites are only found at the extreme 5' and 3' ends of the nucleic acid to facilitate cloning. Preferably all of the above considerations are taken into account when optimising the nucleic acid sequence. The skilled addressee is able to make such modifications to the original FVIII sequence based on prior knowledge in the art in relation to the codon bias of the chosen host and other teachings (*e.g.*Codon bias and heterologous protein expression. Gustafsson C, Govindarajan S, Minshull J. Trends Biotechnol 2004 22:346-53). Certain companies such as Geneart (Regensburg, Germany), GeneScript (Piscataway, New Jersey, USA) and DNA2.0 (Menlo Park, California, USA) provide a service for optimising and synthesising nucleic acid sequences that are tailored for expression in a specified host organism.

Expression vectors useful in the present invention include chromosomal-, episomal- and virus-derived vectors, for example vectors comprising bacterial plasmids, bacteriophages, yeast episomes, yeast chromosomal elements, viruses such as baculoviruses, papova viruses, vaccinia viruses, adenoviruses, fowl pox viruses, pseudorabies viruses and retroviruses, and vectors derived from combinations thereof, such as cosmids and phagemids.

Recombinant expression vectors may be introduced into host cells using well-known techniques such as infection, transduction, transfection, transvection, electroporation and transformation. The vector may be, for example, a phage, plasmid, viral or retroviral vector. Retroviral vectors may be replication competent or replication defective. In the latter case, viral propagation generally will occur only in complementing host cells.

The nucleic acid encoding the FVIII should be operatively linked to an appropriate promoter. Suitable eukaryotic promoters include the cytomegalovirus immediate early promoter, the herpes simplex virus thymidine kinase promoter, the early and late SV40 promoters, the promoters of retroviral long terminal repeats (LTRs), such as those of the Rous sarcoma virus and metallothionein promoters, such as the mouse metallothionein-I promoter. Synthetic promoters are also suitable.

The expression constructs will further contain sites for transcription initiation, termination and, in the transcribed region, a ribosome-binding site for translation. The coding portion of the mature transcripts expressed by the constructs will include a translation-initiating AUG at the beginning and a termination codon appropriately positioned at the end of the nucleic acid sequence to be translated. It is facile, using synthetic genes, to optimise all of these features of the insert to maximise gene-expression levels and recombinant-protein yields.

As indicated, the expression vectors will preferably include at least one selectable marker. Such markers include *e.g.* dihydrofolate reductase or neomycin or zeocin resistance for eukaryotic cell culture and e.g. tetracycline or ampicillin-resistance genes for culturing in *E. coli* and other bacteria.

As indicated, introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals, such as Davis LGG *et al.,* (21).

As indicated, transcription of the DNA encoding the polypeptides of the present invention by higher eukaryotes may be increased by inserting an enhancer sequence into the vector. Enhancers are *cis*-acting elements of DNA, usually from about 10 to about 300 bp that act to increase transcriptional activity of a promoter in a given host cell-type. Examples of enhancers include the SV40 enhancer, which is located on the late side of the replication origin at bp 100 to 270, the cytomegalovirus early-promoter enhancer, the polyoma enhancer on the late side of the replication origin, and adenovirus enhancers.

It will be recognised in the art that the amino-acid residue sequence of a FVIII polypeptide may be selectively varied without having a significantly detrimental effect on the structural integrity or functional properties of the protein. If such differences in sequence are contemplated, it should be remembered that there are regions of the protein that are critical to its biological activity. There will also be residues that are critical to the folding of the protein or for stabilisation of its folded structure. Some residues serve as glycosylation sites, recognised by enzymes that covalently attach glycans to, for example, Asparagine. In general, it may be possible to safely replace residues that contribute directly or indirectly to structure or function by other residues that are chemically similar (this is known as a conservative substitution). In the cases of amino-acid residues that contribute neither to structural integrity nor to functional sites, it may be possible to safely replace such a residue with an amino-acid residue of a different chemical nature (a non-conservative replacement).

Typically seen as conservative substitutions are the replacements, one for another, amongst the aliphatic amino-acids Ala, Val, Leu and Ile; interchange of the hydroxyl residues Ser and Thr; exchange of the acidic residues Asp and Glu; substitution between the amide residues Asn and Gln; exchange of the basic residues Lys and Arg; and replacements amongst the aromatic residues Phe and Tyr. Non-conservative substitutions could include substitutions with both naturally encoded amino-acid residues and a non-naturally encoded (unnatural) amino-acid residue. The unnatural amino-acid residue could be one that serves as a site-specific attachment sites for conjugation with chemical moieties (such as polyethylene glycols (PEGs) and other polymers), or with biochemical groups (such as glycans) that enhance the therapeutic efficacy of FVIII.

As indicated in detail above, further guidance concerning which amino acid changes are likely to be phenotypically silent (*i.e.* are not likely to have a significant deleterious effect on a function) can be found in Bowie (19).

The terms "nucleic acid" or "nucleic acid fragment" refer to any one or more nucleic acid segments, e.g., DNA or RNA fragments, present in a polynucleotide or construct. While the term "nucleic acid," as used herein, is meant to include any nucleic acid, the term "nucleic acid fragment" is used herein to specifically denote a fragment of a designed or synthetic codon optimised coding region encoding a polypeptide, or fragment, variant, or derivative thereof, which has been optimised according to the codon usage of a given species or cell type. As used herein, a "coding region" is a portion of nucleic acid that consists of codons translated into amino acids. Although a "stop codon" (TAG, TGA, or TAA) is not translated into an amino acid, it may be considered to be part of a coding region, but any flanking sequences, for example promoters, ribosome binding sites, transcriptional terminators, and the like, are not part of a coding region. Two or more nucleic acids or nucleic acid fragments of the present invention can be present in a single polynucleotide construct, e.g., on a single plasmid, or in separate polynucleotide constructs, e.g., on separate plasmids. Furthermore, any nucleic acid or nucleic acid fragment may encode a single polypeptide, or may encode more than one polypeptide, e.g., a nucleic acid may encode two or more polypeptides. In addition, a nucleic acid may encode a regulatory element such as a promoter or a transcription terminator, or may encode heterologous coding regions, e.g. specialised elements or motifs, such as a secretory signal peptide or a functional domain.

The terms "fragment," "variant," "derivative" and "analog" when referring to FVIII polypeptides of the present invention include any polypeptides which retain at least some of the functional biological activity of the corresponding native polypeptide. Fragments of FVIII polypeptides of the present invention include proteolytic fragments, deletion fragments and in particular, fragments of FVIII polypeptides which exhibit functional biological activity. Variants of FVIII polypeptides of the present invention include fragments as described above, and also polypeptides with altered amino acid sequences due to amino acid substitutions, deletions, or insertions. Variants may occur naturally, such as an allelic variant. By an "allelic variant" is intended alternate forms of a gene occupying a given locus on a chromosome or genome of an organism or virus. Genes II, Lewin, B., ed., John Wiley & Sons, New York (1985), which is incorporated herein by reference. Variant polypeptides may comprise conservative or non-conservative amino acid substitutions, deletions or additions. Derivatives of FVIII polypeptides of the present invention, include polypeptides which have been altered so as to exhibit, for example, additional features not found in the native polypeptide. Examples include fusion proteins. An analog is another form of a FVIII polypeptide of the present invention. An example is a proprotein which can be activated by cleavage of the proprotein to produce an active mature polypeptide.

As used herein, the term "vector" refers to a construct made up of genetic material (i.e., nucleic acids). Typically the vector may be a plasmid which may contain an origin of replication which is functional in bacterial host cells, e.g., *Eschericha coli*, and selectable markers for detecting bacterial host cells comprising the plasmid. Plasmids of the present invention may include genetic elements as described herein arranged such that an inserted coding sequence can be transcribed and translated in eukaryotic cells. Also, while the plasmid may include a sequence from a viral nucleic acid, such viral sequences normally do not cause the incorporation of the plasmid into a viral particle, and the plasmid is therefore a non-viral vector. In certain embodiments described herein, a plasmid is a closed circular DNA molecule.

The term "expression" generally refers to the biological production of a product encoded by a coding sequence. In most cases a DNA sequence, including the coding sequence, is transcribed to form a messenger-RNA (mRNA). The messenger-RNA is translated to form a polypeptide product which has a relevant biological activity. Also, the process of expression may involve further processing steps to the RNA product of transcription, such as splicing to remove introns, and/or post-translational processing of a polypeptide product.

As used herein, the term "polypeptide" is intended to encompass a singular "polypeptide" as well as plural "polypeptides," and comprises any chain or chains of two or more amino acids. Thus, as used herein, terms including, but not limited to "peptide, "protein," "amino acid chain," or any other term used to refer to a chain or chains of two or more amino acids, are included in the definition of a "polypeptide," and the term "polypeptide" may be used instead of, or interchangeably with any of these terms. The term further includes polypeptides which have undergone post-translational modifications, for example, glycosylation, acetylation, phosphorylation, amidation, derivatization by known protecting/blocking groups, proteolytic cleavage, or modification by non-naturally occurring amino acids.

Also included as polypeptides of the present invention are fragments, derivatives, analogs, or variants of the foregoing polypeptides, and any combination thereof. Polypeptides, and fragments, derivatives, analogs, or variants thereof of the present invention can be biologically functional polypeptides related to FVIII polypeptides, which are used to prevent or treat, i.e., cure, ameliorate, lessen the severity of, or prevent or reduce Haemophilia A.

"Codon optimisation" is defined as modifying a nucleic acid sequence for enhanced expression in the cells of the mammal of interest, e.g. hamster, by replacing at least one, more than one, or a significant number, of codons of the native sequence with codons that are more frequently or most frequently used in the genes of that mammal or cell type. Various species and cell types exhibit particular bias for certain codons of a particular amino acid.

Many organisms display a bias for use of particular codons to code for insertion of a particular amino acid in a growing peptide chain. Codon preference or codon bias, differences in codon usage between organisms, is afforded by degeneracy of the genetic code, and is well documented among many organisms. Codon bias often correlates with the efficiency of translation of messenger RNA (mRNA), which is in turn believed to be dependent on, inter alia, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization.

Given the large number of gene sequences available for a wide variety of animal, plant and microbial species, it is possible to calculate the relative frequencies of codon usage. Codon usage tables are readily available, for example, at the "Codon Usage Database" available at www.kazusa.or.jp/codon/ (visited 19^{th} February 2013), and these tables can be adapted in a number of ways. See Nakamura (20). The codon usage table for human, calculated from GenBank , Flat File Release 160.0 [June 15 2007]. These tables use mRNA nomenclature, and so instead of thymine (T) which is found in DNA, the tables use uracil (U) which is found in RNA. The tables have been adapted so that frequencies are calculated for each amino acid, rather than for all 64 codons.

By utilising these or similar tables, one of ordinary skill in the art can apply the frequencies to any given polypeptide sequence, and produce a nucleic acid fragment of a codon optimised coding region which encodes the polypeptide, but which uses codons more optimal for a given species. Codon optimised coding regions can be designed by various different methods.

In one method, termed "uniform optimisation," a codon usage table is used to find the single most frequent codon used for any given amino acid, and that codon is used each time that particular amino acid appears in the polypeptide sequence. In another method, termed "full-optimization," the actual frequencies of the codons are distributed randomly throughout the coding region. Thus, using this method for optimization, if a hypothetical polypeptide sequence had 100 leucine residues, referring to frequency of usage in the humans, about 7, or 7% of the leucine codons would be UUA, about 13, or 13% of the leucine codons would be UUG, about 13, or 13% of the leucine codons would be CUU, about 20, or 20% of the leucine codons would be CUC, about 7, or 7% of the leucine codons would be CUA, and about 41, or 41% of the leucine codons would be CUG. These frequencies would be distributed randomly throughout the leucine codons in the coding region encoding the hypothetical polypeptide. As will be understood by those of ordinary skill in the art, the distribution of codons in the sequence can vary significantly using this method, however, the sequence always encodes the same polypeptide.

In using the "full-optimisation" method, an entire polypeptide sequence, or fragment, variant, or derivative thereof is codon optimised by any of the methods described herein. Various desired fragments, variants or derivatives are designed, and each is then codon optimised individually. Alternatively, a full-length polypeptide sequence is codon optimised for a given species resulting in a codon optimised coding region encoding the entire polypeptide, and then nucleic acid fragments of the codon optimised coding region, which encode fragments, variants, and derivatives of the polypeptide are made from the original codon optimised coding region. As would be well understood by those of ordinary skill in the art, if codons have been randomly assigned to the full-length coding region based on their frequency of use in a given species, nucleic acid fragments encoding fragments, variants, and derivatives would not necessarily be fully codon optimised for the given species. However, such sequences are still much closer to the codon usage of the desired species than the native codon usage. The advantage of this approach is that synthesizing codon optimised nucleic acid fragments encoding each fragment, variant, and derivative of a given polypeptide, although routine, would be time consuming and would result in significant expense.

As well as *in vitro* applications in order to produce industrial quantities of rFVIII and variants thereof, such as B domain deleted versions for use in therapy, the present invention may also be applied to expression of rFVIII as described herein *in vivo* for potential gene therapy application in haemophilia patients.

### Detailed Description

The present invention will now be further described by way of example and with reference to the figures that show:
Figure 1 (also see SEQ ID NO:3) shows a preferred FVIII B-domain deleted amino acid sequence for use in the present invention;
Figure 2 (also see SEQ ID NO:1) shows a codon optimised nucleic acid encoding the FVIII B-domain deleted amino acid sequence shown in Figure 1, for expression in hamster; and
Figure 3: (a - f) shows the results of experiments concerning cell viability following expression of FVIII alone and in conjunction with VWF. CHO DG44 cells were transfected with the DNA constructs indicated. The percent cell viability of the transfected cells is shown and the control no DNA transfection is shown. Selection (G418 or zeocin) was initiated 48 h after transfection.

### Examples

### Introduction

CHO DG44 cell line is a dihydrofoate reductase (DHFR)-deficient cell line adapted to growth as a suspension culture. p571_BDDFVIIIcoop is an expression vector (11) containing expression cassettes for the *Neo* gene conferring resistance to the antibiotic geneticin (G418); *DHFR* allowing DHFR⁻ cells to grow in the absence of hypoxanthine and thymidine; and BDDFVIIIcoop allowing the expression of human B-domain deleted human FVIII. We have previously transfected CHO cells with the same vector containing non-codon optimised HFVIII and HBDDFVIII sequences and repeatedly obtained stably transfected clonal cell lines, albeit with poor expression characteristics (unpublished and 12, 13). pcDNA3.1Zeo_VWFcoop is based on the expression vector containing expression cassettes for the *Zeo* gene conferring resistance to the antibiotic Zeocin http://www.lifetechnologies.com/1/1/10790-pcdna3-1-zeo-mammalian-expression-vector.html and VWFcoop allowing the expression of human VWF.

### Methods and Materials

### DNA constructs

DNA sequences codon optimised for expression in hamster cells encoding human BDDFVIII and VWF were generated by analysis of the BDDFVIII SQ cDNA sequence (Sanberg et al (22)) and the VWF reference sequence (NP_000543.2) and adaption of the codon usage to the bias of *Cricetulus griseus* using codon adaptation index performed by GeneArt (GeneArt AG) using their in-house proprietary software GeneOptimizer. The nucleic acid sequence of codon optimised versions of human BDD FVIII and VWF for expression in CHO cells are identified in SEQ ID NOs: 1 and 2 respectively. The corresponding amino acid sequences for BDD FVIII and VWF are shown in SEQ ID NOS: 3 and 4 respectively. Optimisation also removed cis-acting sequence motifs, including internal TATA-boxes, chi-sites and ribosomal entry sites, AT- or GC-rich sequence stretches, AU-rich elements, inhibitory and cis-acting repressor sequence elements, repeat sequences, RNA secondary structures, and all cryptic splice sites. The optimised genes BDDFVIIIcoop and VWFcoop were synthesised and cloned into the plasmids p571 (pNeoIG502; Kemball-Cook et al (11) respectively. Each construct was fully sequenced prior to use (Source BioScience UK Ltd).

### Transfection and selection of CHO cells

CHO DG44 cells were purchased from Life Technologies. The cells were maintained in CD DG44 media supplemented with 8 mmol/L glutamine and 0.1% pluronic F-68 (Life Technologies) at 37°C, 5% CO₂ on an orbital shaker (120 rpm). CHO DG44 cells with a viability >95% were used in all transfections. 18 µg of linearised plasmid DNA was used in each transfection. The cells were transfected with the FreeStyle MAX reagent according to the manufacturer's instructions (Life Technologies). Briefly, 18µL of plasmid DNA and 15µL of FreeStyle MAX reagent in OptiPRO SFM media was incubated for 10 minutes at room temperature to allow the formation of DNA-lipid complexes. The complex was then transferred to a 125mL flask containing 30mL of cells (0.5 x 10⁶ cells/mL) and incubated in a 37°C, 5% CO₂ incubator on an orbital shaker (120 rpm). 48 hours after transfection either G418 (250µg/mL) or zeocin (200µg/mL) was added to the culture to select for BDDFVIIIcoop or VWFcoop respectively. Cell counts (total and non-viable) were performed every 3-4 days using a Nucleocounter according to the manufacturer's instructions (Sartorius Stedim).

### PCR analysis

Cells were analysed by PCR using oligonucleotide primers designed to amplify amplicons generating overlapping fragments across the entire coding sequences and the vector cDNA junctions. Briefly, 100 µL of cells (2.97 x 10⁵ cells) were harvested by centrifugation and resuspended in 75 µL of alkaline lysis reagent (25 mM NaOH, 0.2 mM EDTA pH 12), and incubated at 95 °C for 30 minutes before adding 75 µL of neutralizing reagent (40mM TRIS pH 5). PCR reactions were performed using 1 µL of the extracted DNA as template, PCR master mix (Promega), 1µmol/L 5' and 3' oligonucleotide primer pairs (Sigma Genosys) using the PCR program: 95 °C, 3 minutes; 95 °C, 30 seconds, 55 °C, 30 seconds, 72 °C 60 seconds repeated for 30 cycles and finally 72 °C, 10 minutes. PCR fragments were analysed by agarose gel electrophoresis.

### Measurement of FVIII

The cofactor activity of media samples was assessed using the Biophen FVIII:C Chromagenic assay (Quadratech Diagnostics) as per the manufacturer's instructions. A standard curve was constructed by using 1:2 serial dilutions of a plasma calibrator (Biophen; Quadratech Diagnostics). Standards and samples were assayed in duplicate.

### Results

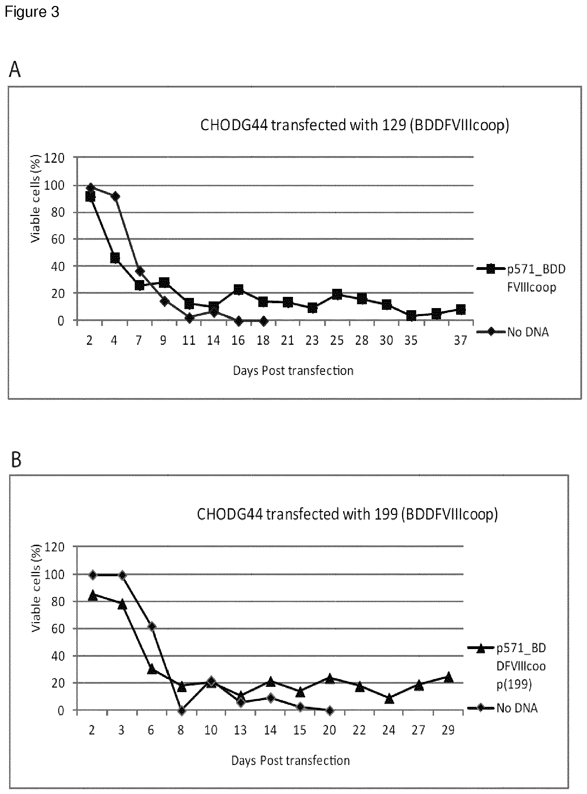

CHO DG44 cells were transfected with linearised plasmid DNAs either (p571_BDDFVIIIcoop or pcDNA3.1Zeo_VWFcoop) and cultured for 48 hours, after which selection for stably transfected cells was initiated by the addition of appropriate antibiotic selection to (G418 (geneticin) for the BDDFVIIIcoop and zeocin for the VWFcoop). Cell counts were performed every 3-4 days to determine cell viability (Figure 3A).

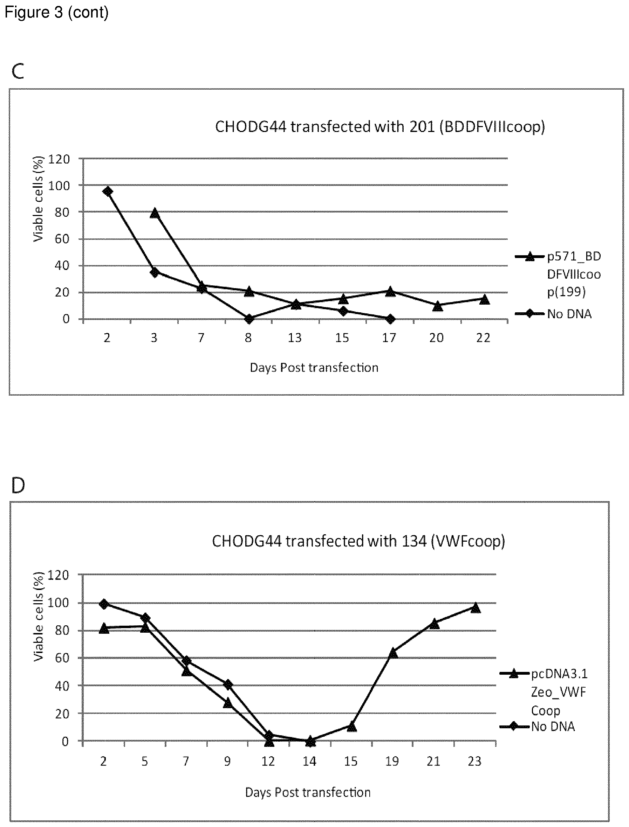

CHO DG44 cells not transfected with the p571_BDDFVIIIcoop were sensitive to G418 and cell viability was zero after 14 days selection as predicted from the results of a G418 sensitivity test performed on non-transfected CHO DG44 cells. The majority of cells in the transfected cell culture with p571_BDDFVIIIcoop were also sensitive to G418 representing either non-transfected cells or cells in which no stable integration has occurred. In comparison with the non-transfected control cell viability of the p571_BDDFVIIIcoop transfected culture remained above zero suggesting the presence of stably transfected cells. However, by day 41 the cell viability dropped to zero (Figure 3A). This experiment was repeated a further two times and on both occasions a similar profile was observed (Figure 3B and 3C). No stable transfectants were obtained on each of these three occasions. DNA prepared from cells 38 days after transfection was positive by PCR for the p571_BDDFVIIIcoop sequence. Hence the CHO DG44 cells were successfully transfected with the expression vector as evidenced by resistance to G418 at a concentration that killed non-transfected cells and PCR positivity for the expression cassette. The failure to obtain stable viable transfectants was unexpected and may be due to the high level of expression of BDDFVIIIcoop leading to apoptosis. CHO DG44 cells not transfected with the pcDNA3.1Zeo_VWFcoop were sensitive to Zeocin and cell viability was zero after 14 days selection as predicted from the results of a Zeocin sensitivity test performed on non-transfected CHO DG44 cells. The majority of cells in the transfected cell culture with pcDNA3.1Zeo_VWFcoop were also sensitive to Zeocin representing either non-transfected cells or cells in which no stable integration has occurred. In comparison with the non-transfected control cell viability of the pcDNA3.1Zeo_VWFcoop transfected culture remained above zero suggesting the presence of stably transfected cells. The cell viability rapidly recovered and by 23 days after the transfection the culture was 97% viable (Figure 3D). Viable growing cultures of these cells were established and shown by PCR to contain the entire VWFcoop expression cassette.

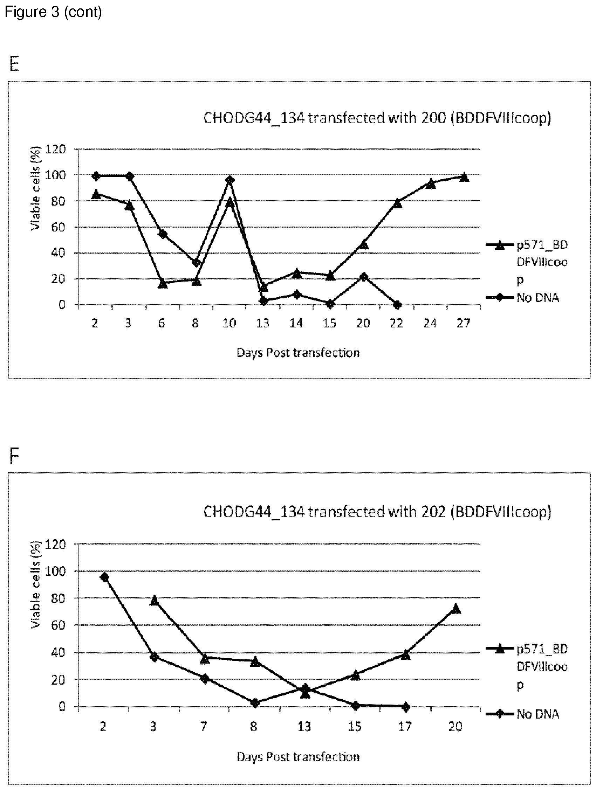

The CHO DG44 cells stably transfected with pcDNA3.1Zeo_VWFcoop were then transfected with p571_BDDFVIIIcoop. CHO DG44 cells not transfected with the p571_BDDFVIIIcoop were sensitive to G418 and cell viability was zero after 15-17 days selection as predicted from the results of a G418 sensitivity test performed on non-transfected CHO DG44 cells. The majority of cells in the transfected cell culture with p571_BDDFVIIIcoop were also sensitive to G418 representing either non-transfected cells or cells in which no stable integration has occurred. In comparison with the non-transfected control cell and previous transfections of CHO DG44 with p571_BDDFVIIIcoop the CHO DG44_pcDNA3.1Zeo_VWFcoop culture transfected with p571_BDDFVIIIcoop remained above zero suggesting the presence of stably transfected cells. The cell viability rapidly recovered and by 27 days after transfection the culture was >97% viable (Figure 3E). This transfection was repeated and similar results obtained (Figure 3F). In both these cases viable growing cultures of these cells were established and shown by PCR to contain the entire BDDFVIIIcoop and VWFCOOP expression cassettes. These polyclonal cell populations were subsequently determined to be expressing an average level of 0.003pg/cell/day prior to methotrexate amplification. Activity levels of produced material were also analysed (using the FVIII:C chromogenic assay and Plasma calibrator from Biophen) revealing that the majority of produced material was biologically active.

These polyclonal cells were then subjected to a clonal selection procedure consisting of :
- Preparing a 30 mL culture of cells to a cell count of 1 x 10⁶ cells/mL.
- Diluting the cells by serial 10-fold dilution to a concentration of < 0.05 cells/mL in complete OptiCHO media (LifeTech).
- Plating 0.2 mL of these cells in sterile 96-Well Edge Plates, (Thermo Scientific Nunc)
- Medium evaporation from the perimeter wells of a microtitre plate is a significant problem, especially when the cells are grown for >3 days. As the medium evaporates over time (even in a humidified environment) the concentration of media components changes, influencing cell growth and performance. A traditional approach to this problem has been to fill the perimeter wells with sterile water or PBS. An alternative is the use of Nunc Edge 96-well plates, which have a reservoir surrounding the outer area of the plate that can be filled with sterile water, preventing evaporation from the inner wells. In this case all 96 wells can be used.
- Plates were then incubated undisturbed for 10-14 days in a 37 °C, 5% CO2 incubator with no shaking.
- Growing cells were transferred from the 96-well plates to 24-well plates by pipetting the cells up and down gently and transferring the entire contents of one well to a single well of a 24-well plate containing 1 mL of fresh complete OptiCHO media
- Media was then assayed for FVIII antigen and activity by standard procedures.
- mycoplasma testing was undertaken throughout.

This procedure resulted in the isolation of three cell lines expressing rhFVIII at 0.03-4pg/cell/day.

Polyclonal populations of cells derived from this process were then subjected to methotrexate amplification (starting at 0.2µM MTX and increasing by 0.1µM at each stage) to increase the copy number of the rhFVIII expression cassette. Cell productivity was assayed throughout by the above means. Cells amplified to a level of 0.5µM MTX increased productivity of rhFVIII. Beyond this level of MTX, rhFVIII fell dramatically. Polyclonal cells derived from this stage expressed rhFVIII at ≥0.1 pg/cell/day.

These polyclonal cells can be subjected to a clonal selection procedure as described above. This will result in the isolation of cell lines expressing rhFVIII at an increased rate of perhaps 0.3-0.4 pg/cell/day.

### REFERENCES

1. Zhang et al Nat. Genet. (2003) 34: 220-225
2. Zhang et al J. Biol. Chem. (2005) 280: 25881-25886
3. Pipe, Haemophilia (2009) 15:1187-1196
4. Pittman, et al Blood (1994) 84: 4214-4225
5. Malhotra et al, Proc. Natl. Acad. Sci. USA (2008) 105: 18525-18530
6. Kaufman et al, J. Biol. Chem. (1988) 263: 6352-6362 and USJ, 198, 349
7. Miao et al, Blood (2004) 103: 3412-3419
8. Ward et al,,Blood (2011) 117:798-807
9. Marin, Biotechnol J. (2008) 3: 1047-1057
10. Tsai et al, J. Mol. Biol. (2008) 383: 281-291
11. Kemball-Cook et al, Gene (1994) 139: 275-279
12. David et a,l J Thromb Haemost (2003) 1: 139-146
13. David et al, B.J. Haem. (2001)113;604-615
14. Wise et al, JBiolChem (1991)266, 21948-21955
15. Kolind et al, JBiotec (2011) 151, 357-362)
16. Spencer et al, Molecular Therapy (2011), 19/2, 302-309.
17. Kaufman and Sharp, J. Mol. Biol. (1982)159, 601-621)
18. Lorimer D, Raymond A, Walchli J, Mixon M, Barrow A, Wallace E, Grice R, Burgin A, Stewart L., BMC Biotechnol. 2009 9:36)
19. Bowie, et al., "Deciphering the Message in Protein Sequences: Tolerance to Amino Acid Substitutions," Science 247:1306-1310 (1990).
20. Nakamura, Y., et al. "Codon usage tabulated from the international DNA sequence databases: status for the year 2000" Nucl. Acids Res. 28:292 (2000).
21. Davis LGG et al., Basic Methods in Molecular Biology, (2nd Ed., McGraw-Hill, 1995).
22. Sanberg et al (2001) Thromb Haemost 85, 93-100.

## Claims

1. A mammalian cell comprising an exogenously introduced nucleic acid which is capable of recombinantly expressing FVIII wherein the nucleic acid, or portion thereof which encodes said FVIII has been codon optimised for expression by the mammalian cell; and wherein the cell further recombinantly co-expresses von Willebrand Factor (VWF).

2. The mammalian cell according to claim 1 wherein the nucleic acid encoding the VWF has also been codon optimised for expression by the mammalian cell.

3. The mammalian cell according to claims 1 or 2 wherein the mammalian FVIII is human FVIII or a B domain deleted version of human FVIII.

4. The mammalian cell according to any preceding claim wherein the cell is a 293, CHO, 3T3, BHK, MICK, Vero, Junket, or HeLa cell, as well as derivatives of such cells known in the art.

5. The mammalian cell according to any preceding claim wherein the mammalian FVIII is human FVIII or a B domain deleted version of human FVIII codon optimised for hamster and the mammalian cell is a CHO or BHK cell.

6. The mammalian cell according to any of claims 3-5 wherein the FVIII amino acid sequence is a FVIII sequence from which the B domain has been deleted, and optionally replaced with a short B-domain spacer rich in asparagine-linked oligosaccharides.

7. The mammalian cell according to any preceding claim wherein the FVIII amino acid sequence is the sequence shown in SEQ ID NO:1.

8. The mammalian cell according to any preceding claim wherein the nucleic acid sequence encoding the mammalian FVIII is at least 90%, 95%, 96%, 97%, 98% 99%, or is identical to the sequence is shown in SEQ ID NO:3.

9. The mammalian cell according to any preceding claim wherein the VWF amino acid sequence is the sequence shown in SEQ ID NO:2.

10. The mammalian cell according to any preceding claim wherein the VWF amino acid sequence is the sequence shown in SEQ ID NO:4.

11. A cell culture comprising a mammalian cell according to any preceding claim, which is capable of expressing the FVIII in an amount of ≥ 0.1pg/cell/day, typically ≥ 0.25 pg/cell/day.

12. A method of preparing recombinant mammalian FVIII or a B domain deleted version of FVIII, comprising providing a cell, transfecting the cell with a vector capable of recombinantly expressing VWF and thereafter further transfecting cells transfected with VWF with a vector capable of recombinantly expressing FVIII or a B domain deleted version of FVIII, wherein the transfections are carried out with vectors which comprise codon optimised nucleic acid constructs capable of expressing FVIII or a B domain deleted version of FVIII and VWF in said cell.

13. The method according to claim 12 comprising adding methotrexate to the cell culture medium, at a concentration up to 0.5µM.

14. The method according to any of claims 12-13 further comprising purifying FVIII or a B domain deleted version of FVIII or complex comprising FVIII or a B domain deleted version of FVIII from the cell and/or culture medium in which the cell is grown.

## Patentansprüche

1. Säugetierzelle, umfassend eine exogen eingeführte Nucleinsäure, die in der Lage ist, FVIII rekombinant zu exprimieren, wobei die Nucleinsäure oder ein Teil von ihr, der das FVIII codiert, für die Expression durch die Säugetierzelle Codon-optimiert wurde; und wobei die Zelle ferner Von Willebrand-Faktor (VWF) co-exprimiert.

2. Säugertierzelle nach Anspruch 1, wobei die Nucleinsäure, die den VWF codiert, ebenfalls für die Expression durch die Säugetierzelle Codon-optimiert wurde.

3. Säugetierzelle nach Anspruch 1 oder 2, wobei der Säugetier-FVIII ein humaner FVIII oder eine B-Domäne-deletierte Version von humanem FVIII ist.

4. Säugetierzelle nach einem der vorhergehenden Ansprüche, wobei die Zelle eine 293-, CHO-, 3T3-, BHK-, MICK-, Vero-, Junket- oder HeLa-Zelle sowie Derivate derartiger auf dem Fachgebiet bekannter Zellen ist.

5. Säugetierzelle nach einem der vorhergehenden Ansprüche, wobei der Säugetier-FVIII ein humaner FVIII ist oder eine B-Domänen-deletierte Version von humanem FVIII Codon-optimiert für Hamster, und die Säugetierzelle eine CHO- oder BHK-Zelle ist.

6. Säugetierzelle nach einem der Ansprüche 3 bis 5, wobei die FVIII-Aminosäuresequenz eine FVIII-Sequenz ist, von der die B-Domäne deletiert worden ist und wahlweise durch einen kurzen B-Domänen-Spacer ersetzt wurde, der reich an Asparagin-verknüpften Oligosacchariden ist.

7. Säugetierzelle nach einem der vorhergehenden Ansprüche, wobei die FVIII-Aminosäuresequenz die in SEQ ID NO: 1 gezeigte Sequenz ist.

8. Säugetierzelle nach einem der vorhergehenden Ansprüche, wobei die Nucleinsäuresequenz, die den Säugetier-FVIII codiert, zu mindestens 90%, 95%, 96%, 97%, 98%, 99% oder identisch ist mit der Sequenz, die in SEQ ID NO: 3 gezeigt ist.

9. Säugetierzelle nach einem der vorhergehenden Ansprüche, wobei die VWF-Aminosäuresequenz die in SEQ ID NO: 2 gezeigte Sequenz ist.

10. Säugetierzelle nach einem der vorhergehenden Ansprüche, wobei die VWF-Aminosäuresequenz die in SEQ ID NO: 4 gezeigte Sequenz ist.

11. Zellkultur, umfassend eine Säugetierzelle nach einem der vorhergehenden Ansprüche, die in der Lage ist, den FVIII in einer Menge von >0,1 pg/Zelle/Tag, im typischen Fall >0,25 pg/Zelle/Tag, zu exprimieren.

12. Verfahren zur Herstellung von rekombinantem Säuger-FVIII oder einer B-Domäne-deletierten Version von FVIII, umfassend das Bereitstellen einer Zelle, Transfektieren der Zelle mit einem Vektor, der zum rekombinanten Exprimieren von VWF in der Lage ist, und danach weiteres Transfektieren von Zellen, die mit VFIII transfiziert sind mit einem Vektor, der zum rekombinanten Exprimieren von VFIII in der Lage ist oder einer B-Domäne-deletierten Version von FVIII, wobei die Transfektionen mit Vektoren ausgeführt werden, die Codon-optimierte Nucleinsäure-Konstrukte aufweisen, die zum Exprimieren von FVIII oder einer B-Domäne-deletierten Version von FVIII und VWF in der Zelle in der Lage sind.

13. Verfahren nach Anspruch 12, umfassend die Zugabe von Methotrexat zu dem Zellkulturmedium in einer Konzentration von bis zu 0,5 µl.

14. Verfahren nach einem der Ansprüche 12 bis 13, ferner umfassend das Reinigen von FVIII oder einer B-Domäne-deletierten Version von FVIII oder einem Komplex, der FVIII oder eine B-Domäne-deletierte Version von FVIII aus der Zelle und/oder dem Kulturmedium aufweist, in dem die Zelle aufgezogen worden ist.

## Revendications

1. Cellule mammifère comprenant un acide nucléique introduit de façon exogène qui est capable d'exprimer par recombinaison FVIII, où l'acide nucléique, ou une portion de celui-ci, qui code pour ledit FVIII a été optimisé sur les codons pour une expression par la cellule mammifère; et où la cellule en outre co-exprime par recombinaison le facteur de von Willebrand (VWF).

2. Cellule mammifère selon la revendication 1, dans laquelle l'acide nucléique codant pour le VWF a été également optimisé sur les codons pour une expression par la cellule mammifère.

3. Cellule mammifère selon les revendications 1 ou 2, dans laquelle le FVIII mammifère est un FVIII humain ou une version à domaine B délété d'un FVIII humain.

4. Cellule mammifère selon l'une quelconque des revendications précédentes, où la cellule est une cellule 293, CHO, 3T3, BHK, MICK, Vero, Junket ou HeLa, aussi bien que des dérivés de ces cellules connus de la technique.

5. Cellule mammifère selon l'une quelconque des revendications précédentes, dans laquelle le FVIII mammifère est un FVIII humain ou une version à domaine B délété d'un FVIII humain à codons optimisés pour un hamster et la cellule mammifère est une cellule CHO ou BHK.

6. Cellule mammifère selon l'une quelconque des revendications 3-5, dans laquelle la séquence d'acides aminés de FVIII est une séquence de FVIII à partir de laquelle le domaine B a été délété et optionnellement remplacé avec un espaceur de domaine B court riche en oligosaccharides liés à une asparagine.

7. Cellule mammifère selon l'une quelconque des revendications précédentes, dans laquelle la séquence d'acides aminés de FVIII est la séquence montrée dans la SEQ ID NO: 1.

8. Cellule mammifère selon l'une quelconque des revendications précédentes, dans laquelle la séquence d'acide nucléique codant pour le FVIII mammifère est au moins 90%, 95%, 96%, 97%, 98%, 99% ou est identique à la séquence montrée dans la SEQ ID NO:3.

9. Cellule mammifère selon l'une quelconque des revendications précédentes, dans laquelle la séquence d'acides aminés de VWF est la séquence montrée dans la SEQ ID NO:2.

10. Cellule mammifère selon l'une quelconque des revendications précédentes, dans laquelle la séquence d'acides aminés de VWF est la séquence montrée dans la SEQ ID NO:4.

11. Culture cellulaire comprenant une cellule mammifère selon l'une quelconque des revendications précédentes, qui est capable d'exprimer le FVIII dans une quantité ≥ 0,1 pg/cellule/jour, typiquement ≥ 0,25 pg/cellule/jour.

12. Méthode pour la préparation d'un FVIII mammifère recombinant ou d'une version à domaine B délété de FVIII, comprenant la fourniture d'une cellule, la transfection de la cellule avec un vecteur capable d'exprimer par recombinaison VWF et par la suite en outre la transfection des cellules transfectées avec VWF avec un vecteur capable d'exprimer par recombinaison FVIII ou une version à domaine B délété de FVIII, dans laquelle les transfections sont réalisées avec des vecteurs qui comprennent des constructions d'acides nucléiques à codons optimisés capables d'exprimer FVIII ou une version à domaine délété B de FVIII et VWF dans ladite cellule.

13. Méthode selon la revendication 12, comprenant l'ajout de méthotrexate au milieu de culture cellulaire, à une concentration jusqu'à 0,5 µm.

14. Méthode selon l'une quelconque des revendications 12-13, comprenant en outre la purification de FVIII ou d'une version à domaine B délété de FVIII ou d'un complexe comprenant FVIII ou une version à domaine B délété de FVIII à partir de la cellule et/ou du milieu de culture dans lequel la cellule est mise à croître.
